# EUROPEAN PATENT APPLICATION

(11) **EP 1 745 801 A1**
(43) Date of publication of application: **24.01.2007**
(21) Application number: 05737308.6
(22) Date of filing: 28.04.2005
(51) Int. Cl.: A61K 45/00, A61K 38/55, A61P 35/00, A61P 43/00, C12Q 1/48, G01N 33/15, G01N 33/50, C12N 9/99, C12N 15/09

(54) **METHOD OF INHIBITING TELOMERASE ACTIVITY AND INHIBITOR**

(30) Priority: 13.05.2004 JP 2004143902
(71) Applicant: DAIICHI PHARMACEUTICAL CO., LTD., Chuo-ku, Tokyo 103-8234 (JP)
(72) Inventor: WADA, Naoya, Daiichi Pharmaceutical Co., Ltd., Edogawa-ku, Tokyo 134-8630 (JP); OKAMOTO, Takashi, Daiichi Pharmaceutical Co., Ltd., Edogawa-ku, Tokyo 134-8630 (JP); TANIGAKI, Keiji, 6310061 (JP); DOI, Hirofumi, Celestar Lexico-Sciences, Inc., Mihama-ku, Chiba-shi, Chiba 2618501 (JP); KITUCHI, Y., Celestar Lexico-Sciences, Inc., Mihama-ku, Chiba-shi, Chiba 2618501 (JP); IMAI, K., Celestar Lexico-Sciences, Inc., Mihama-ku, Chiba-shi, Chiba 2618501 (JP)
(74) Representative: De Clercq, Ann G. Y.
(86) International application number: PCT/JP2005/008239
(87) International publication number: WO 2005/110476

(57) **Abstract**

The present invention provides a method for inhibiting the activation of telomerase and an agent for inhibiting the activation of telomerase, a method for inhibiting telomerase activity and an agent for inhibiting telomerase activity, a method for preventing and/or a method for treating a cancer disease, an agent for preventing and/or an agent for treating a cancer disease, all of which comprises inhibiting the binding of MAPKAPK3 to TERT that is a catalytic subunit of telomerase or inhibiting the phosphorylation of TERT by active MAPKAPK3; a method of identifying a compound that inhibits the binding of MAPKAPK3 to TERT or a compound that inhibits the phosphorylation of TERT by active MAPKAPK3; and a reagent kit.

SEQUENCE LISTING

<110>  DAIICHI PHARMACEUTICAL CO., LTD.

<120>  Method of inhibiting telomerase activity and inhibitor

<130>  DAII-003-EP

<140>  05737308.6
<141>  2005-04-28

<150>  JP P2004-143902
<151>  2004-05-13

<160>  13

<170>  PatentIn version 3.1

<210>  1
<211>  1149
<212>  DNA
<213>  Homo sapiens

<220>
<221>  CDS
<222>  (1)..(1149)
<223>

<220>
<221>  misc_feature
<223>  A polynucleotide encoding MAPKAPK3 (SEQ ID NO:2)

<400>  1
atg gat ggt gaa aca gca gag gag cag ggg ggc cct gtg ccc ccg cca      48
Met Asp Gly Glu Thr Ala Glu Glu Gln Gly Gly Pro Val Pro Pro Pro
1               5                   10                  15

gtt gca ccc ggc gga ccc ggc ttg ggc ggt gct ccg ggg ggg cgg cgg      96
Val Ala Pro Gly Gly Pro Gly Leu Gly Gly Ala Pro Gly Gly Arg Arg
            20                  25                  30

gag ccc aag aag tac gca gtg acc gac gac tac cag ttg tcc aag cag      144
Glu Pro Lys Lys Tyr Ala Val Thr Asp Asp Tyr Gln Leu Ser Lys Gln
        35                  40                  45

gtg ctg ggc ctg ggt gtg aac ggc aaa gtg ctg gag tgc ttc cat cgg      192
Val Leu Gly Leu Gly Val Asn Gly Lys Val Leu Glu Cys Phe His Arg
    50                  55                  60

cgc act gga cag aag tgt gcc ctg aag ctc ctg tat gac agc ccc aag      240
Arg Thr Gly Gln Lys Cys Ala Leu Lys Leu Leu Tyr Asp Ser Pro Lys
65                  70                  75                  80

gcc cgg cag gag gta gac cat cac tgg cag gct tct ggc ggc ccc cat      288
Ala Arg Gln Glu Val Asp His His Trp Gln Ala Ser Gly Gly Pro His
                85                  90                  95

att gtc tgc atc ctg gat gtg tat gag aac atg cac cat ggc aag cgc      336
Ile Val Cys Ile Leu Asp Val Tyr Glu Asn Met His His Gly Lys Arg
            100                 105                 110

tgt ctc ctc atc atc atg gaa tgc atg gaa ggt ggt gag ttg ttc agc      384
Cys Leu Leu Ile Ile Met Glu Cys Met Glu Gly Gly Glu Leu Phe Ser
        115                 120                 125

agg att cag gag cgt ggc gac cag gct ttc act gag aga gaa gct gca      432
Arg Ile Gln Glu Arg Gly Asp Gln Ala Phe Thr Glu Arg Glu Ala Ala
    130                 135                 140

gag ata atg cgg gat att ggc act gcc atc cag ttt ctg cac agc cat      480
Glu Ile Met Arg Asp Ile Gly Thr Ala Ile Gln Phe Leu His Ser His
145                 150                 155                 160

aac att gcc cac cga gat gtc aag cct gaa aac cta ctc tac aca tct      528
Asn Ile Ala His Arg Asp Val Lys Pro Glu Asn Leu Leu Tyr Thr Ser

                165                 170                 175

aag gag aaa gac gca gtg ctt aag ctc acc gat ttt ggc ttt gct aag 576
Lys Glu Lys Asp Ala Val Leu Lys Leu Thr Asp Phe Gly Phe Ala Lys
            180                 185                 190

gag acc acc caa aat gcc ctg cag aca ccc tgc tat act ccc tat tat 624
Glu Thr Thr Gln Asn Ala Leu Gln Thr Pro Cys Tyr Thr Pro Tyr Tyr
        195                 200                 205

gtg gcc cct gag gtc ctg ggt cca gag aag tat gac aag tca tgt gac 672
Val Ala Pro Glu Val Leu Gly Pro Glu Lys Tyr Asp Lys Ser Cys Asp
    210                 215                 220

atg tgg tcc ctg ggt gtc atc atg tac atc ctc ctt tgt ggc ttc cca 720
Met Trp Ser Leu Gly Val Ile Met Tyr Ile Leu Leu Cys Gly Phe Pro
225                 230                 235                 240

ccc ttc tac tcc aac acg ggc cag gcc atc tcc ccg ggg atg aag agg 768
Pro Phe Tyr Ser Asn Thr Gly Gln Ala Ile Ser Pro Gly Met Lys Arg
                245                 250                 255

agg att cgc ctg ggc cag tac ggc ttc ccc aat cct gag tgg tca gaa 816
Arg Ile Arg Leu Gly Gln Tyr Gly Phe Pro Asn Pro Glu Trp Ser Glu
            260                 265                 270

gtc tct gag gat gcc aag cag ctg atc cgc ctc ctg ttg aag aca gac 864
Val Ser Glu Asp Ala Lys Gln Leu Ile Arg Leu Leu Leu Lys Thr Asp
        275                 280                 285

ccc aca gag agg ctg acc atc act cag ttc atg aac cac ccc tgg atc 912
Pro Thr Glu Arg Leu Thr Ile Thr Gln Phe Met Asn His Pro Trp Ile
    290                 295                 300

aac caa tcg atg gta gtg cca cag acc cca ctc cac acg gcc cga gtg 960
Asn Gln Ser Met Val Val Pro Gln Thr Pro Leu His Thr Ala Arg Val
305                 310                 315                 320

ctg cag gag gac aaa gac cac tgg gac gaa gtc aag gag gag atg acc 1008
Leu Gln Glu Asp Lys Asp His Trp Asp Glu Val Lys Glu Glu Met Thr
                325                 330                 335

agt gcc ttg gcc act atg cgg gta gac tac gac cag gtg aag atc aag 1056
Ser Ala Leu Ala Thr Met Arg Val Asp Tyr Asp Gln Val Lys Ile Lys
            340                 345                 350

gac ctg aag acc tct aac aac cgg ctc ctc aac aag agg aga aaa aag 1104
Asp Leu Lys Thr Ser Asn Asn Arg Leu Leu Asn Lys Arg Arg Lys Lys
        355                 360                 365

cag gca ggc agc tcc tct gcc tca cag ggc tgc aac aac cag tag 1149
Gln Ala Gly Ser Ser Ser Ala Ser Gln Gly Cys Asn Asn Gln
    370                 375                 380

<210> 2
<211> 382
<212> PRT
<213> Homo sapiens

<400> 2

Met Asp Gly Glu Thr Ala Glu Glu Gln Gly Gly Pro Val Pro Pro Pro
1               5                   10                  15

Val Ala Pro Gly Gly Pro Gly Leu Gly Gly Ala Pro Gly Gly Arg Arg
            20                  25                  30

Glu Pro Lys Lys Tyr Ala Val Thr Asp Asp Tyr Gln Leu Ser Lys Gln
        35                  40                  45

Val Leu Gly Leu Gly Val Asn Gly Lys Val Leu Glu Cys Phe His Arg
    50                  55                  60

Arg Thr Gly Gln Lys Cys Ala Leu Lys Leu Leu Tyr Asp Ser Pro Lys
65                  70                  75                  80

Ala Arg Gln Glu Val Asp His His Trp Gln Ala Ser Gly Gly Pro His
                85                  90                  95

Ile Val Cys Ile Leu Asp Val Tyr Glu Asn Met His His Gly Lys Arg
            100                 105                 110

Cys Leu Leu Ile Ile Met Glu Cys Met Glu Gly Gly Glu Leu Phe Ser
        115                 120                 125

Arg Ile Gln Glu Arg Gly Asp Gln Ala Phe Thr Glu Arg Glu Ala Ala
    130                 135                 140

Glu Ile Met Arg Asp Ile Gly Thr Ala Ile Gln Phe Leu His Ser His
145                 150                 155                 160

Asn Ile Ala His Arg Asp Val Lys Pro Glu Asn Leu Leu Tyr Thr Ser
                165                 170                 175

Lys Glu Lys Asp Ala Val Leu Lys Leu Thr Asp Phe Gly Phe Ala Lys
            180                 185                 190

Glu Thr Thr Gln Asn Ala Leu Gln Thr Pro Cys Tyr Thr Pro Tyr Tyr
        195                 200                 205

Val Ala Pro Glu Val Leu Gly Pro Glu Lys Tyr Asp Lys Ser Cys Asp
    210                 215                 220

Met Trp Ser Leu Gly Val Ile Met Tyr Ile Leu Leu Cys Gly Phe Pro
225                 230                 235                 240

Pro Phe Tyr Ser Asn Thr Gly Gln Ala Ile Ser Pro Gly Met Lys Arg
                245                 250                 255

Arg Ile Arg Leu Gly Gln Tyr Gly Phe Pro Asn Pro Glu Trp Ser Glu
            260                 265                 270

Val Ser Glu Asp Ala Lys Gln Leu Ile Arg Leu Leu Leu Lys Thr Asp
        275                 280                 285

Pro Thr Glu Arg Leu Thr Ile Thr Gln Phe Met Asn His Pro Trp Ile
    290                 295                 300

Asn Gln Ser Met Val Val Pro Gln Thr Pro Leu His Thr Ala Arg Val
305                 310                 315                 320

Leu Gln Glu Asp Lys Asp His Trp Asp Glu Val Lys Glu Glu Met Thr
                325                 330                 335

Ser Ala Leu Ala Thr Met Arg Val Asp Tyr Asp Gln Val Lys Ile Lys
            340                 345                 350

Asp Leu Lys Thr Ser Asn Asn Arg Leu Leu Asn Lys Arg Arg Lys Lys
        355                 360                 365

Gln Ala Gly Ser Ser Ser Ala Ser Gln Gly Cys Asn Asn Gln
370                 375                 380

<210>  3
<211>  3399
<212>  DNA
<213>  Homo sapiens

<220>
<221>  CDS
<222>  (1)..(3399)
<223>

<220>
<221>  misc_feature
<223>  A polynucleotide encoding TERT (SEQ ID NO:4)

<400>  3
atg ccg cgc gct ccc cgc tgc cga gcc gtg cgc tcc ctg ctg cgc agc       48
cac tac cgc gag gtg ctg ccg ctg gcc acg ttc gtg cgg cgc ctg ggg       96
ccc cag ggc tgg cgg ctg gtg cag cgc ggg gac ccg gcg gct ttc cgc      144
gcg ctg gtg gcc cag tgc ctg gtg tgc gtg ccc tgg gac gca cgg ccg      192
ccc ccc gcc gcc ccc tcc ttc cgc cag gtg tcc tgc ctg aag gag ctg      240
gtg gcc cga gtg ctg cag agg ctg tgc gag cgc ggc gcg aag aac gtg      288
ctg gcc ttc ggc ttc gcg ctg ctg gac ggg gcc cgc ggg ggc ccc ccc      336
gag gcc ttc acc acc agc gtg cgc agc tac ctg ccc aac acg gtg acc      384
gac gca ctg cgg ggg agc ggg gcg tgg ggg ctg ctg ctg cgc cgc gtg      432
ggc gac gac gtg ctg gtt cac ctg ctg gca cgc tgc gcg ctc ttt gtg      480
ctg gtg gct ccc agc tgc gcc tac cag gtg tgc ggg ccg ccg ctg tac      528
cag ctc ggc gct gcc act cag gcc cgg ccc ccg cca cac gct agt gga      576
ccc cga agg cgt ctg gga tgc gaa cgg gcc tgg aac cat agc gtc agg      624
gag gcc ggg gtc ccc ctg ggc ctg cca gcc ccg ggt gcg agg agg cgc      672
ggg ggc agt gcc agc cga agt ctg ccg ttg ccc aag agg ccc agg cgt     720
ggc gct gcc cct gag ccg gag cgg acg ccc gtt ggg cag ggg tcc tgg     768
gcc cac ccg ggc agg acg cgt gga ccg agt gac cgt ggt ttc tgt gtg     816
gtg tca cct gcc aga ccc gcc gaa gaa gcc acc tct ttg gag ggt gcg     864
ctc tct ggc acg cgc cac tcc cac cca tcc gtg ggc cgc cag cac cac     912
gcg ggc ccc cca tcc aca tcg cgg cca cca cgt ccc tgg gac acg cct     960
tgt ccc ccg gtg tac gcc gag acc aag cac ttc ctc tac tcc tca ggc     1008
gac aag gag cag ctg cgg ccc tcc ttc cta ctc agc tct ctg agg ccc     1056
agc ctg act ggc gct cgg agg ctc gtg gag acc atc ttt ctg ggt tcc     1104
agg ccc tgg atg cca ggg act ccc cgc agg ttg ccc cgc ctg ccc cag     1152
cgc tac tgg caa atg cgg ccc ctg ttt ctg gag ctg ctt ggg aac cac     1200
gcg cag tgc ccc tac ggg gtg ctc ctc aag acg cac tgc ccg ctg cga     1248
gct gcg gtc acc cca gca gcc ggt gtc tgt gcc cgg gag aag ccc cag     1296
ggc tct gtg gcg gcc ccc gag gag gag gac aca gac ccc cgt cgc ctg     1344
gtg cag ctg ctc cgc cag cac agc agc ccc tgg cag gtg tac ggc ttc     1392
gtg cgg gcc tgc ctg cgc cgg ctg gtg ccc cca ggc ctc tgg ggc tcc     1440
agg cac aac gaa cgc cgc ttc ctc agg aac acc aag aag ttc atc tcc     1488
ctg ggg aag cat gcc aag ctc tcg ctg cag gag ctg acg tgg aag atg     1536
agc gtg cgg gac tgc gct tgg ctg cgc agg agc cca ggg gtt ggc tgt     1584
gtt ccg gcc gca gag cac cgt ctg cgt gag gag atc ctg gcc aag ttc 1632
ctg cac tgg ctg atg agt gtg tac gtc gtc gag ctg ctc agg tct ttc 1680
ttt tat gtc acg gag acc acg ttt caa aag aac agg ctc ttt ttc tac 1728
cgg aag agt gtc tgg agc aag ttg caa agc att gga atc aga cag cac 1776
ttg aag agg gtg cag ctg cgg gag ctg tcg gaa gca gag gtc agg cag 1824
cat cgg gaa gcc agg ccc gcc ctg ctg acg tcc aga ctc cgc ttc atc 1872
ccc aag cct gac ggg ctg cgg ccg att gtg aac atg gac tac gtc gtg 1920
gga gcc aga acg ttc cgc aga gaa aag agg gcc gag cgt ctc acc tcg 1968
agg gtg aag gca ctg ttc agc gtg ctc aac tac gag cgg gcg cgg cgc 2016
ccc ggc ctc ctg ggc gcc tct gtg ctg ggc ctg gac gat atc cac agg 2064
gcc tgg cgc acc ttc gtg ctg cgt gtg cgg gcc cag gac ccg ccg cct 2112
gag ctg tac ttt gtc aag gtg gat gtg acg ggc gcg tac gac acc atc 2160
ccc cag gac agg ctc acg gag gtc atc gcc agc atc atc aaa ccc cag 2208
aac acg tac tgc gtg cgt cgg tat gcc gtg gtc cag aag gcc gcc cat 2256
ggg cac gtc cgc aag gcc ttc aag agc cac gtc tct acc ttg aca gac 2304
ctc cag ccg tac atg cga cag ttc gtg gct cac ctg cag gag acc agc 2352
ccg ctg agg gat gcc gtc gtc atc gag cag agc tcc tcc ctg aat gag 2400
gcc agc agt ggc ctc ttc gac gtc ttc cta cgc ttc atg tgc cac cac 2448
gcc gtg cgc atc agg ggc aag tcc tac gtc cag tgc cag ggg atc ccg 2496
cag ggc tcc atc ctc tcc acg ctg ctc tgc agc ctg tgc tac ggc gac 2544
atg gag aac aag ctg ttt gcg ggg att cgg cgg gac ggg ctg ctc ctg 2592
cgt ttg gtg gat gat ttc ttg ttg gtg aca cct cac ctc acc cac gcg 2640
aaa acc ttc ctc agg acc ctg gtc cga ggt gtc cct gag tat ggc tgc 2688
gtg gtg aac ttg cgg aag aca gtg gtg aac ttc cct gta gaa gac gag 2736
gcc ctg ggt ggc acg gct ttt gtt cag atg ccg gcc cac ggc cta ttc 2784
ccc tgg tgc ggc ctg ctg ctg gat acc cgg acc ctg gag gtg cag agc 2832
gac tac tcc agc tat gcc cgg acc tcc atc aga gcc agt ctc acc ttc 2880
aac cgc ggc ttc aag gct ggg agg aac atg cgt cgc aaa ctc ttt ggg 2928
gtc ttg cgg ctg aag tgt cac agc ctg ttt ctg gat ttg cag gtg aac 2976
agc ctc cag acg gtg tgc acc aac atc tac aag atc ctc ctg ctg cag 3024
gcg tac agg ttt cac gca tgt gtg ctg cag ctc cca ttt cat cag 3069
caa gtt tgg aag aac ccc aca ttt ttc ctg cgc gtc atc tct gac 3114
acg gcc tcc ctc tgc tac tcc atc ctg aaa gcc aag aac gca ggg 3159
atg tcg ctg ggg gcc aag ggc gcc gcc ggc cct ctg ccc tcc gag 3204
gcc gtg cag tgg ctg tgc cac caa gca ttc ctg ctc aag ctg act 3249
cga cac cgt gtc acc tac gtg cca ctc ctg ggg tca ctc agg aca 3294
gcc cag acg cag ctg agt cgg aag ctc ccg ggg acg acg ctg act 3339
gcc ctg gag gcc gca gcc aac ccg gca ctg ccc tca gac ttc aag 3384
acc atc  ctg gac tga                                                    3399

<210>  4
<211>  1132
<212>  PRT
<213>  Homo sapiens

<400>  4

<210>  5
<211>  1149

<212>  DNA
<213>  Artificial

<220>
<223>  A polynucleotide encoding an inactive variant of MAPKAPK3 (SEQ ID
        NO:2) which amino acid residues at positions 201 and 313 are bot
       h replaced to alanine from threonine

<400>  5
atggatggtg aaacagcaga ggagcagggg ggccctgtgc ccccgccagt tgcacccggc      60

ggacccggct tgggcggtgc tccggggggg cggcgggagc ccaagaagta cgcagtgacc     120

gacgactacc agttgtccaa gcaggtgctg ggcctgggtg tgaacggcaa agtgctggag     180

tgcttccatc ggcgcactgg acagaagtgt gccctgaagc tcctgtatga cagccccaag     240

gcccggcagg aggtagacca tcactggcag gcttctggcg gcccccatat tgtctgcatc     300

ctggatgtgt atgagaacat gcaccatggc aagcgctgtc tcctcatcat catggaatgc     360

atggaaggtg gtgagttgtt cagcaggatt caggagcgtg gcgaccaggc tttcactgag     420

agagaagctg cagagataat gcgggatatt ggcactgcca tccagtttct gcacagccat     480

aacattgccc accgagatgt caagcctgaa aacctactct acacatctaa ggagaaagac     540

gcagtgctta agctcaccga ttttggcttt gctaaggaga ccacccaaaa tgccctgcag     600

gccccctgct atactcccta ttatgtggcc cctgaggtcc tgggtccaga gaagtatgac     660

aagtcatgtg acatgtggtc cctgggtgtc atcatgtaca tcctcctttg tggcttccca     720

cccttctact ccaacacggg ccaggccatc tccccgggga tgaagaggag gattcgcctg     780

ggccagtacg gcttccccaa tcctgagtgg tcagaagtct ctgaggatgc caagcagctg     840

atccgcctcc tgttgaagac agaccccaca gagaggctga ccatcactca gttcatgaac     900

cacccctgga tcaaccaatc gatggtagtg ccacaggccc cactccacac ggcccgagtg     960

ctgcaggagg acaaagacca ctgggacgaa gtcaaggagg agatgaccag tgccttggcc    1020

actatgcggg tagactacga ccaggtgaag atcaaggacc tgaagacctc taacaaccgg    1080

ctcctcaaca agaggagaaa aaagcaggca ggcagctcct ctgcctcaca gggctgcaac    1140

aaccagtag                                                            1149

<210>  6
<211>  382
<212>  PRT
<213>  Artificial

<220>
<223>  An inactive variant of MAPKAPK3 (SEQ ID NO:2) which amino acid re
       sidues at positions 201 and 313 are both replaced to alanine from
        threonine

<400>  6

Met Asp Gly Glu Thr Ala Glu Glu Gln Gly Gly Pro Val Pro Pro Pro
1               5                   10                  15

Val Ala Pro Gly Gly Pro Gly Leu Gly Gly Ala Pro Gly Gly Arg Arg
            20                  25                  30

Glu Pro Lys Lys Tyr Ala Val Thr Asp Asp Tyr Gln Leu Ser Lys Gln
        35                  40                  45

Val Leu Gly Leu Gly Val Asn Gly Lys Val Leu Glu Cys Phe His Arg
    50                  55                  60

Arg Thr Gly Gln Lys Cys Ala Leu Lys Leu Leu Tyr Asp Ser Pro Lys
65                  70                  75                  80

Ala Arg Gln Glu Val Asp His His Trp Gln Ala Ser Gly Gly Pro His
                85                  90                  95

Ile Val Cys Ile Leu Asp Val Tyr Glu Asn Met His His Gly Lys Arg
            100                 105                 110

Cys Leu Leu Ile Ile Met Glu Cys Met Glu Gly Gly Glu Leu Phe Ser
        115                 120                 125

Arg Ile Gln Glu Arg Gly Asp Gln Ala Phe Thr Glu Arg Glu Ala Ala
    130                 135                 140

Glu Ile Met Arg Asp Ile Gly Thr Ala Ile Gln Phe Leu His Ser His
145                 150                 155                 160

Asn Ile Ala His Arg Asp Val Lys Pro Glu Asn Leu Leu Tyr Thr Ser
                165                 170                 175

Lys Glu Lys Asp Ala Val Leu Lys Leu Thr Asp Phe Gly Phe Ala Lys
            180                 185                 190

Glu Thr Thr Gln Asn Ala Leu Gln Ala Pro Cys Tyr Thr Pro Tyr Tyr
        195                 200                 205

Val Ala Pro Glu Val Leu Gly Pro Glu Lys Tyr Asp Lys Ser Cys Asp
    210                 215                 220

Met Trp Ser Leu Gly Val Ile Met Tyr Ile Leu Leu Cys Gly Phe Pro
225                 230                 235                 240

Pro Phe Tyr Ser Asn Thr Gly Gln Ala Ile Ser Pro Gly Met Lys Arg
                245                 250                 255

Arg Ile Arg Leu Gly Gln Tyr Gly Phe Pro Asn Pro Glu Trp Ser Glu
            260                 265                 270

Val Ser Glu Asp Ala Lys Gln Leu Ile Arg Leu Leu Leu Lys Thr Asp
        275                 280                 285

Pro Thr Glu Arg Leu Thr Ile Thr Gln Phe Met Asn His Pro Trp Ile
    290                 295                 300

Asn Gln Ser Met Val Val Pro Gln Ala Pro Leu His Thr Ala Arg Val
305                 310                 315                 320

Leu Gln Glu Asp Lys Asp His Trp Asp Glu Val Lys Glu Glu Met Thr
                325                 330                 335

Ser Ala Leu Ala Thr Met Arg Val Asp Tyr Asp Gln Val Lys Ile Lys
            340                 345                 350

Asp Leu Lys Thr Ser Asn Asn Arg Leu Leu Asn Lys Arg Arg Lys Lys
        355                 360                 365

Gln Ala Gly Ser Ser Ser Ala Ser Gln Gly Cys Asn Asn Gln
    370                 375                 380

<210>  7
<211>  1149
<212>  DNA
<213>  Artificial

<220>
<223>  A polynucleotide encoding an active variant of MAPKAPK3 (SEQ ID N
       O:2) which amino acid residues at positions 201 and 313 are both
       replaced to glutamic acid from threonine

<400>  7
atggatggtg aaacagcaga ggagcagggg ggccctgtgc ccccgccagt tgcacccggc      60

ggacccggct tgggcggtgc tccggggggg cggcgggagc ccaagaagta cgcagtgacc     120

gacgactacc agttgtccaa gcaggtgctg ggcctgggtg tgaacggcaa agtgctggag     180

tgcttccatc ggcgcactgg acagaagtgt gccctgaagc tcctgtatga cagccccaag     240

gcccggcagg aggtagacca tcactggcag gcttctggcg gcccccatat tgtctgcatc     300

ctggatgtgt atgagaacat gcaccatggc aagcgctgtc tcctcatcat catggaatgc     360

atggaaggtg gtgagttgtt cagcaggatt caggagcgtg gcgaccaggc tttcactgag     420

agagaagctg cagagataat gcgggatatt ggcactgcca tccagtttct gcacagccat     480

aacattgccc accgagatgt caagcctgaa aacctactct acacatctaa ggagaaagac     540

gcagtgctta agctcaccga ttttggcttt gctaaggaga ccacccaaaa tgccctgcag     600

gagccctgct atactcccta ttatgtggcc cctgaggtcc tgggtccaga gaagtatgac     660

aagtcatgtg acatgtggtc cctgggtgtc atcatgtaca tcctcctttg tggcttccca     720

cccttctact ccaacacggg ccaggccatc tccccgggga tgaagaggag gattcgcctg     780

ggccagtacg gcttccccaa tcctgagtgg tcagaagtct ctgaggatgc caagcagctg     840

atccgcctcc tgttgaagac agaccccaca gagaggctga ccatcactca gttcatgaac     900

cacccctgga tcaaccaatc gatggtagtg ccacaggagc cactccacac ggcccgagtg     960

ctgcaggagg acaaagacca ctgggacgaa gtcaaggagg agatgaccag tgccttggcc    1020

actatgcggg tagactacga ccaggtgaag atcaaggacc tgaagacctc taacaaccgg    1080

ctcctcaaca agaggagaaa aaagcaggca ggcagctcct ctgcctcaca gggctgcaac    1140

aaccagtag                                                            1149

<210>  8
<211>  382
<212>  PRT
<213>  Artificial

<220>
<223>  An active variant of MAPKAPK3 (SEQ ID NO:2) which amino acid resi
       dues at positions 201 and 313 are both replaced to glutamic acid
       from threonine

<400>  8

Met Asp Gly Glu Thr Ala Glu Glu Gln Gly Gly Pro Val Pro Pro Pro
1               5                   10                  15

Val Ala Pro Gly Gly Pro Gly Leu Gly Gly Ala Pro Gly Gly Arg Arg
            20                  25                  30

Glu Pro Lys Lys Tyr Ala Val Thr Asp Asp Tyr Gln Leu Ser Lys Gln
        35                  40                  45

Val Leu Gly Leu Gly Val Asn Gly Lys Val Leu Glu Cys Phe His Arg
    50                  55                  60

Arg Thr Gly Gln Lys Cys Ala Leu Lys Leu Leu Tyr Asp Ser Pro Lys
65                  70                  75                  80

Ala Arg Gln Glu Val Asp His His Trp Gln Ala Ser Gly Gly Pro His
                85                  90                  95

Ile Val Cys Ile Leu Asp Val Tyr Glu Asn Met His His Gly Lys Arg
            100                 105                 110

Cys Leu Leu Ile Ile Met Glu Cys Met Glu Gly Gly Glu Leu Phe Ser
        115                 120                 125

Arg Ile Gln Glu Arg Gly Asp Gln Ala Phe Thr Glu Arg Glu Ala Ala
    130                 135                 140

Glu Ile Met Arg Asp Ile Gly Thr Ala Ile Gln Phe Leu His Ser His
145                 150                 155                 160

Asn Ile Ala His Arg Asp Val Lys Pro Glu Asn Leu Leu Tyr Thr Ser
                165                 170                 175

Lys Glu Lys Asp Ala Val Leu Lys Leu Thr Asp Phe Gly Phe Ala Lys
            180                 185                 190

Glu Thr Thr Gln Asn Ala Leu Gln Glu Pro Cys Tyr Thr Pro Tyr Tyr
        195                 200                 205

Val Ala Pro Glu Val Leu Gly Pro Glu Lys Tyr Asp Lys Ser Cys Asp
    210                 215                 220

Met Trp Ser Leu Gly Val Ile Met Tyr Ile Leu Leu Cys Gly Phe Pro
225                 230                 235                 240

Pro Phe Tyr Ser Asn Thr Gly Gln Ala Ile Ser Pro Gly Met Lys Arg
                245                 250                 255

Arg Ile Arg Leu Gly Gln Tyr Gly Phe Pro Asn Pro Glu Trp Ser Glu
            260                 265                 270

Val Ser Glu Asp Ala Lys Gln Leu Ile Arg Leu Leu Leu Lys Thr Asp
        275                 280                 285

Pro Thr Glu Arg Leu Thr Ile Thr Gln Phe Met Asn His Pro Trp Ile
    290                 295                 300

Asn Gln Ser Met Val Val Pro Gln Glu Pro Leu His Thr Ala Arg Val
305                 310                 315                 320

Leu Gln Glu Asp Lys Asp His Trp Asp Glu Val Lys Glu Glu Met Thr
                325                 330                 335

Ser Ala Leu Ala Thr Met Arg Val Asp Tyr Asp Gln Val Lys Ile Lys
            340                 345                 350

Asp Leu Lys Thr Ser Asn Asn Arg Leu Leu Asn Lys Arg Arg Lys Lys
        355                 360                 365

Gln Ala Gly Ser Ser Ser Ala Ser Gln Gly Cys Asn Asn Gln
    370                 375                 380

<210>  9
<211>  6
<212>  PRT
<213>  Artificial

<220>
<223>  Partial sequence of TERT (SEQ ID NO:4) , which is highly homologo
       us to that of MAPKAPK3 (SEQ ID NO:2)

<400>  9

Pro Pro Pro Ala Ala Pro
1               5

<210>  10
<211>  6
<212>  PRT
<213>  Artificial

<220>
<223>  Partial sequence of MAPKAPK3 (SEQ ID NO:2)  , which is highly hom
       ologous to that of TERT (SEQ ID NO:4)

<400>  10

Pro Pro Pro Val Ala Pro
1               5

<210>  11
<211>  6
<212>  PRT
<213>  Artificial

<220>
<223>  Partial sequence of TERT (SEQ ID NO:4) , which is highly homologo
       us to that of MAPKAPK3 (SEQ ID NO:2)

<400>  11

Ala Pro Gly Ala Arg Arg
1               5

<210>  12
<211>  6
<212>  PRT
<213>  Artificial

<220>
<223>  Partial sequence of MAPKAPK3 (SEQ ID NO:2)  , which is highly hom
       ologous to that of TERT (SEQ ID NO:4)

<400>  12

Ala Pro Gly Gly Arg Arg
1               5

<210>  13
<211>  5

<212>  PRT
<213>  Artificial

<220>
<223>  Partial sequence identical in the sequences of TERT (SEQ ID NO:4)
       and MAPKAPK3 (SEQ ID NO:2)

<400>  13

Ala Arg Val Leu Gln
1               5

## Description

### TECHNICAL FIELD

The present invention relates to a method for inhibiting the activation oftelomerase, a method for inhibiting telomerase activity, an agent for inhibiting the activation oftelomerase, and an agent for inhibiting telomerase activity, comprising inhibiting the binding of MAPKAPK3 (mitogen-activated protein kinase-activated protein kinase-3) to telomerase reverse transcriptase (TERT), or inhibiting the phosphorylation of TERT by active MAPKAPK3.
Moreover, the present invention relates to a method for inhibiting telomerase activity comprising using an inactive variant of MAPKAPK3, and an agent for inhibiting telomerase activity comprising the variant.
Moreover, the present invention relates to a method of identifying a compound that inhibits the binding of MAPKAPK3 to TERT, or a compound which inhibits the phosphorylation of TERT by active MAPKAPK3.
Furthermore, the present invention relates to a method for preventing and/or a method for treating a disease attributable to the enhanced telomerase activity, comprising using the foregoing method for inhibiting the activation oftelomerase and/or the foregoing method for inhibiting telomerase activity.
Furthermore, the present invention relates to an agent for preventing and/or an agent for treating a disease attributable to the enhanced telomerase activity, comprising inhibiting the activation of telomerase using the foregoing method for inhibiting the activation oftelomerase and/or inhibiting telomerase activity using the foregoing method for inhibiting telomerase activity.
Moreover, the present invention relates to a reagent kit comprising at least one of MAPKAPK3, a polynucleotide encoding MAPKAPK3 and a vector containing the polynucleotide and a transformant containing the vector; and at least one of TERT, a polynucleotide encoding TERT, a vector containing the polynucleotide and a transformant containing the vector.

### BACKGROUND ART

Telomerase is an enzyme for catalyzing the extension reaction of telomere sequence at chromosome ends in eukaryotic cells, and is a ribonucleic protein comprising telomerase RNA (TR) that is a template RNA molecule and TERT that is a catalytic subunit (Non-Patent Reference 1).

Telomerase gives the immortality (unlimited lifespan) to a cell by maintaining telomere length. Telomere is a simple repeated sequence located in the terminal portion of chromosome DNA in a eukaryotic cell, and contributes to maintaining the stability of chromosome. A usual DNA polymerase is responsible for the replication of DNA upon cell division, but does not replicate completely to the extreme end of DNA, thereby to generate a shortened telomere. A normal somatic cell repeats the cell divisions at predetermined times and stops the proliferation to fall in cell senescence or death.

Telomerase activity is not detected in a normal cell except a germ cell, various kinds of stem cells and a lymphocyte in peripheral blood. Therefore, most of normal cells have a limited lifespan, since a telomere length is more and more shortened every cell division and can not be maintained.

On the other hand, most of immortalized cells such as a cancer cell have the detectable telomerase activity. Namely, it is considered that extension of telomere, having been shortened by cell division, resulting in maintained telomere length is one of mechanisms by which a cancer cell acquires ability for unlimited proliferation. It is apparent that telomerase allows telomeres to maintain the length to facilitate the immortalization of cancer cells, by the report showing that elimination of telomerase activity in cancer cells with antisense RNA made the cancer cells stop the cell proliferation after ten or more times of cell divisions to fall in death. Moreover, it has been reported that a dominant negative type telomerase that was expressed within a cancer cell induced elimination of telomerase activity, shortened telomere length in accordance with cell division, appearance of fused chromosome ends and cell death.

Activation of telomerase has been reported, such as the activation of telomerase by activation of TERT gene expression, and the activation of telomerase via phosphorylation of TERT. As for the former, most of normal cells are determined to express TR gene, but not determined to express TERT gene, while many cancer cells are observed to express both TR and TERT genes. Therefore, it is considered that a cell with telomerase activity detected, such as a cancer cell, uses a certain mechanism to activate TERT gene to express. As for the latter, it has been reported that telomerase activity in the nuclear extract from a cancer cell was reduced by dephosphorylation treatment, which suggests a possibility that phophorylation is involved in the activation of telomerase (Non-Patent Reference 2). Moreover, it has been reported that the telomerase activity in nuclear extract from a cancer cell was enhanced by the reaction of the nuclear extract with Akt or Protein kinase C (PKC), which suggests a possibility that these kinases are involved in the activation of telomerase (Non-Patent References 3 and 4). Furthermore, it has been reported that excessive expression of active Akt in human umbilical vein endothelial cell resulted in enhanced telomerase activity in the cell (Non-Patent References 5 and 6). Furthermore, it has been reported that excessive expression of dominant negative type Akt in human umbilical vein endothelial cell resulted in reduced telomerase activity in the cell (Non-Patent References 5 and 6). Furthermore, it has been also reported that PI3-kinase inhibitor reduces the telomerase activity in human umbilical vein endothelial cell (Non-Patent References 5 and 6). However, as for Akt, it has been reported that no difference is observed in Akt activity between in a breast cancer tissue and in the normal tissue (Non-Patent Reference 7). Moreover, as for PKC, it is unclear whether PKC enhances telomerase activity in a cancer cell. The PKC alpha, one of PKC isozymes, and Akt show low homology to MAPKAPK3 at a level of primary structure, which are about 14.89%, and 13.76%, respectively. The homology of the other PKC isozymes to MAPKAPK3 is low at a level of primary structure, as well.

MAPKAPK3 is one of serine/threonine kinases, and has been reported that it is phosphorylated by a MAP kinase family (such as ERK, p38, JNK) to activate. As for the function of MAPKAPK3, it has been only reported that it is involved in luteinization mechanism. Moreover, it has been reported that MAPKAPK3 phosphorylates substrates such as HSP27 (small heat shock protein 27), a transcription factor CREB (cAMP regulatory element binding protein) and a transcription factor E47. However, there has been no report on the relationship between these substrates and telomerase.

The References cited in the specification are listed as follows:
Patent Reference 1: "Intematinal Publicaition No. WO01/67299 pamphlet".
Non-patent Reference 1: Kelleher C. et al., "TRENDS in Biochemical Sciences", 2002, Vol.27, p.572-579.
Non-patent Reference 2: "Journal of Biological Chemistry", 1997, Vol.272, p.16729-16732.
Non-patent Reference 3: "Journal of Biological Chemistry", 1999, Vol.274, p. 13085-13090.
Non-patent Reference 4: "Journal of Biological Chemistry", 1998, Vol.273, p.33436-33442.
Non-patent Reference 5: "FEBS Letters", 2001, Vol.493, p.21-25.
Non-patent Reference 6: "FEBS Letters", 2003, Vol.536, p.180-186.
Non-patent Reference 7: "International Journal of Cancer", 2002, Vol.98, p.148-154.
Non-patent Reference 8: "American Journal of Respiratory Cell and Molecular Biology", 2002, Vol.26, p.558-564.
Non-patent Reference 9: "Cancer Letters", 2003, Vol.191, p.229-237.
Non-patent Reference 10: "Journal of Clinical Investigation", 1997, Vol.99, p.1478-1483.
Non-patent Reference 11: "Cancer Research", 1995, Vol.55, p.4182-4187.
Non-patent Reference 12: "Journal of Clinical Investigation", 1997, Vol.99, p.1463-1464.
Non-patent Reference 13: "Blood", 1999, Vol.93, p.3893-3899.
Non-patent Reference 14: "American Journal of Pathology", 1998, Vol.153, p.1411-1423.
Non-patent Reference 15: "Journal of Steroid Biochemistry and Molecular Biology", 2002, Vol.80, p.239-256.
Non-patent Reference 16: "Pathology, Research and Practice", 2000, Vol.196, p.817-826.
Non-patent Reference 17: "Pathology Oncology Research", 2001, Vol.7, p.171-177.
Non-patent Reference 18: "Cancer Research", 1999, Vol.59, p.279-284.
Non-patent Reference 19: "Anticancer Research", 2001, Vol.21, p.2733-2738.
Non-patent Reference 20: "Cancer Research", 2001, Vol.61, p.6500-6510.
Non-patent Reference 21: "Hepatology", 1998, Vol.27, p.951-958.
Non-patent Reference 22: "Journal of Urology", 1999, Vol.162, p.1537-1542.
Non-patent Reference 23: Ulmer K.M., "Science", 1983 Vol. 219, p.666-671.
Non-patent Reference 24: "PEPUTIDO GOUSEI", Maruzen Co., Ltd., 1975.
Non-patent Reference 25: "Peptide Synthesis", Interscience, New York, 1996.
Non-patent Reference 26: "Molecular and Cellular Biology", 1996, Vol. 16, p.6687-6697.
Non-patent Reference 27: Kim N.W. et al., "Science", 1994, Vol.266, No.5193, p.2011-2015.
Non-patent Reference 28: Wenz C. et al., "The EMBO Journal", 2001, Vol.20, No.13, p.3526-3534.
Non-patent Reference 29: Tatematsu K. et al., "Oncogene", 1996, Vol.13, No. 10, p.2265-2274.
Non-patent Reference 30: Nakamura Y. et al., "Clinical Chemistry", 1999, Vol.45, No.10, p.1718-1724.
Non-patent Reference 31: Maesawa C. et al., "Nucleic Acids Research", 2003, Vol.31, No.2, p.E4-4.
Non-patent Reference 32: "Biotechniques", 2002, Vol.32, No. 5, p. 1154-1156, 1158, 1160.
Non-patent Reference 33: Paddison P.J. et al., "Genes and Development", 2002, Vol. 16, p.948-958.

### DISCLOSURE OF THE INVENTION

### [PROBLEM TO BE SOLVED BY THE INVENTION]

An object of the present invention is to find out and provide a protein involved in the activation oftelomerase. Moreover, the object of the present invention includes providing a means for inhibiting the activation oftelomerase and a means for inhibiting telomerase activity. Moreover, the object of the present invention includes providing a useful means for preventing and/or treating a disease attributable to the enhanced telomerase activity, such as cancer.

### [MEANS FOR SOLVING THE PROBLEM]

The present inventors have concentrated intensively efforts to solve the aforementioned object, and predicted *in-silico* that TERT, which is a catalytic subunit oftelomerase, interacts with MAPKAPK3 (mitogen-activated protein kinase-activated protein kinase-3). Then, the present inventors have demonstrated that MAPKAPK3 is involved in the activation of telomerase. Concretely, the present inventors have demonstrated that MAPKAPK3 binds to TERT, and that telomerase activity is enhanced in a dose dependent manner of active MAPKAPK3. From these findings, the present inventors believe that MAPKAPK3 binds to TERT, and is subsequently phosphorylated by ERK, JNK and/or p38 or the like to become active MAPKAPK3, which then phosphorylates the TERT to enhance the telomerase activity.

In the present invention, it has been also found that inactive MAPKAPK3, which binds to TERT, can inhibit the telomerase activity. Concretely, it has been demonstrated that the inactive MAPKAPK3 binds to TERT, and that the inactive MAPKAPK3 that binds to TERT reduces the telomerase activity, resulting in a shortened telomere length of genomic DNA. From these findings, the present inventors believe that the inactive MAPKAPK3, which binds to TERT, inhibits telomerase activation and/or telomerase, resulting in inhibited telomerase activity. The inactive MAPKAPK3 that binds to TERT has a reduced or eliminated kinase activity compared with MAPKAPK3 and active MAPKAPK3. However, the inactive MAPKAPK3 that binds to TERT has an ability to bind to TERT that is a substrate of MAPKAPK3. Therefore, it is considered that the inactive MAPKAPK3 that binds to TERT competitively inhibits the binding of MAPKAPK3 to TERT, thereby to inhibit the activation oftelomerase, which results in inhibited telomerase activity. In this way, the inactive MAPKAPK3 that binds to TERT can inhibit telomerase activity.

As described above, to inhibit the binding of MAPKAPK3 to TERT, or to inhibit the phosphorylation of telomerase by active MAPKAPK3 can inhibit the activation oftelomerase, which results in inhibited telomerase activity.

Moreover, inactive MAPKAPK3 can be used to reduce telomerase activity, resulting in a shortened telomere length. Therefore, to inhibit the binding of MAPKAPK3 to TERT, or to inhibit the phosphorylation oftelomerase by active MAPKAPK3 can reduce telomerase activity, resulting in a shortened telomere length, thereby to bring a cancer cell to have a limited lifespan and a lowered stability in chromosome, which induces cell death. Thus, it can be achieved to prevent and/or treat a cancer disease such as breast cancer.

The present invention has been thus achieved.

That is, one aspect of the present invention relates to a method for inhibiting the activation of telomerase, which is selected from the following group:
(i) a method for inhibiting the activation of telomerase, comprising inhibiting the binding of MAPKAPK3 (mitogen-activated protein kinase-activated protein kinase-3) to TERT (telomerase reverse transcriptase); and
(ii) a method for inhibiting the activation of telomerase, comprising inhibiting the phosphorylation of TERT by active MAPKAPK3.

Another aspect of the present invention relates to a method for inhibiting the activation of telomerase, comprising inhibiting the binding of MAPKAPK3 (mitogen-activated protein kinase-activated protein kinase-3) to TERT (telomerase reverse transcriptase).

A further aspect of the present invention relates to a method for inhibiting telomerase activity, comprising using the aforementioned method for inhibiting the activation of telomerase.

A still further aspect of the present invention relates to a method for inhibiting telomerase activity by an inactive variant of MAPKAPK3 (mitogen-activated protein kinase-activated protein kinase-3), wherein the variant is a variant that binds to TERT (telomerase reverse transcriptase).

A further aspect of the present invention relates the aforementioned method for inhibiting telomerase activity, wherein the inactive variant of MAPKAPK3 (mitogen-activated protein kinase-activated protein kinase-3) is a protein shown by the amino acid sequence set forth in SEQ ID NO: 6 in the sequence listing.

A still further aspect of the present invention relates to a method for preventing and/or treating a disease attributable to the enhanced telomerase activity, comprising using the aforementioned method for inhibiting the activation of telomerase and/or the aforementioned method for inhibiting telomerase activity.

A still further aspect of the present invention relates to the aforementioned method for preventing and/or treating a disease attributable to the enhanced telomerase activity, wherein the disease attributable to the enhanced telomerase activity is a cancer disease.

A further aspect of the present invention relates to the aforementioned method for preventing and/or treating a disease attributable to the enhanced telomerase activity, wherein the cancer disease is any of breast cancer, renal cell carcinoma, acute leukemia, glia cell carcinoma, prostatic cancer, neuroepithelial carcinoma, squamous cell carcinoma, liver cell carcinoma, prostatic cancer, and non-small cell lung cancer.

A still further aspect of the present invention relates to the aforementioned method for preventing and/or treating a disease attributable to the enhanced telomerase activity, wherein the cancer disease is a breast cancer disease.

A further aspect of the present invention relates to a method of identifying a compound that inhibits the binding of MAPKAPK3 (mitogen-activated protein kinase-activated protein kinase-3) to TERT (telomerase reverse transcriptase), wherein the method comprises contacting a compound with MAPKAPK3 and/or TERT under a condition allowing the compound to interact with MAPKAPK3 and/or the TERT, introducing a system using a signal and/or a marker that is generated by the binding of MAPKAPK3 to TERT, and detecting the presence, absence or change of the signal and/or the marker, thereby determining whether the compound inhibits the binding of MAPKAPK3 to the TERT.

A still further aspect of the present invention relates to a method of identifying a compound that inhibits the phosphorylation of TERT (telomerase reverse transcriptase) by active MAPKAPK3 (mitogen-activated protein kinase-activated protein kinase-3), wherein the method comprises contacting a compound with active MAPKAPK3 and/or TERT under a condition allowing the compound to interact with active MAPKAPK3 and/or TERT, introducing a system using a signal and/or a marker that is generated by the phosphorylation of TERT by active MAPKAPK3, detecting the presence, absence or change of the signal and/or the marker, thereby determining whether the compound inhibits the phosphorylation of TERT by active MAPKAPK3.

A further aspect of the present invention relates to an agent for inhibiting the activation of telomerase, which is selected from the following group:
(i) an agent for inhibiting the activation oftelomerase, comprising inhibiting the binding of MAPKAPK3 (mitogen-activated protein kinase-activated protein kinase-3) to TERT (telomerase reverse transcriptase); and
(ii) an agent for inhibiting the activation oftelomerase, comprising inhibiting the phosphorylation of TERT by active MAPKAPK3.

A still further aspect of the present invention relates to an agent for inhibiting the activation oftelomerase, which is selected from the following group:
(i) an agent for inhibiting the activation oftelomerase, comprising at least one compound that inhibits the binding of MAPKAPK3 (mitogen-activated protein kinase-activated protein kinase-3) to TERT (telomerase reverse transcriptase); and
(ii) an agent for inhibiting the activation oftelomerase, comprising at least one compound that inhibits the phosphorylation of TERT by active MAPKAPK3.

A further aspect of the present invention relates to an agent for inhibiting telomerase activity, which is selected from the following group:
(i) an agent for inhibiting telomerase activity, comprising inhibiting the binding of MAPKAPK3 (mitogen-activated protein kinase-activated protein kinase-3) to TERT (telomerase reverse transcriptase); and
(ii) an agent for inhibiting telomerase activity, comprising inhibiting the phosphorylation of TERT by active MAPKAPK3.

A still further aspect of the present invention relates to an agent for inhibiting telomerase activity, which is selected from the following group:
(i) an agent for inhibiting telomerase activity, comprising at least one compound that inhibits the binding of MAPKAPK3 (mitogen-activated protein kinase-activated protein kinase-3) to TERT (telomerase reverse transcriptase); and
(ii) an agent for inhibiting telomerase activity, comprising at least one compound that inhibits the phosphorylation of TERT by active MAPKAPK3.

A further aspect of the present invention relates to an agent for inhibiting telomerase activity comprising an inactive variant of MAPKAPK3 (mitogen-activated protein kinase-activated protein kinase-3), wherein the variant is a variant that binds to TERT (telomerase reverse transcriptase).

A still further aspect of the present invention relates to the aforementioned agent for inhibiting telomerase activity comprising an inactive variant of MAPKAPK3 (mitogen-activated protein kinase-activated protein kinase-3), wherein the inactive variant of MAPKAPK3 is a protein shown by the amino acid sequence set forth in SEQ ID NO: 6 in the sequence listing.

A further aspect of the present invention relates to an agent for preventing and/or treating a disease attributable to the enhanced telomerase activity, comprising using the aforementioned method for inhibiting the activation of telomerase and/or the aforementioned method for inhibiting telomerase activity.

A still further aspect of the present invention relates to an agent for preventing and/or treating a disease attributable to the enhanced telomerase activity, comprising at least one of the aforementioned agent for inhibiting the activation of telomerase and the aforementioned agent for inhibiting telomerase activity.

A further aspect of the present invention relates to the aforementioned agent for preventing and/or treating a disease attributable to the enhanced telomerase activity, wherein the disease attributable to the enhanced telomerase activity is a cancer disease.

A still further aspect of the present invention relates to the aforementioned agent for preventing and/or treating a disease attributable to the enhanced telomerase activity, wherein the cancer disease is any of breast cancer, renal cell carcinoma, acute leukemia, glia cell carcinoma, prostatic cancer, neuroepithelial carcinoma, squamous cell carcinoma, liver cell carcinoma, prostatic cancer, and non-small cell lung cancer.

A further aspect of the present invention relates to the aforementioned agent for preventing and/or treating a disease attributable to the enhanced telomerase activity, wherein the cancer disease is a breast cancer disease.

A still further aspect of the present invention relates to a reagent kit, comprising at least one selected from the group consisting of MAPKAPK3 (mitogen-activated protein kinase-activated protein kinase-3), a polynucleotide encoding MAPKAPK3, a vector containing the polynucleotide, and a transformant containing the vector; and at least one selected from the group consisting of TERT (telomerase reverse transcriptase), a polynucleotide encoding TERT, a vector containing the polynucleotide, and a transformant containing the vector.

### [ADVANTAGE OF THE INVENTION]

The present invention can provide a method for inhibiting the activation of telomerase, a method for inhibiting telomerase activity, an agent for inhibiting the activation of telomerase and an agent for inhibiting telomerase activity, each of which comprises inhibiting the binding of MAPKAPK3 to TERT, or inhibiting the phosphorylation of TERT by active MAPKAPK3. Moreover, a method for inhibiting telomerase activity, comprising using an inactive variant of MAPKAPK3, and an agent for inhibiting telomerase activity comprising the inactive variant can be provided. Furthermore, a method of identifying a compound that inhibit the binding of MAPKAPK3 to TERT or a compound that inhibit the phosphorylation of TERT by active MAPKAPK3 can be provided. Moreover, a method for preventing and/or treating a disease attributable to the enhanced telomerase activity comprising using the foregoing method for inhibiting the activation of telomerase and/or using the foregoing method for inhibiting telomerase activity can be provided. Furthermore, an agent for preventing and/or treating a disease attributable to the enhanced telomerase activity comprising inhibiting the activation of telomerase using the foregoing method for inhibiting the activation of telomerase and/or the inhibition of telomerase activity using the foregoing method for inhibiting telomerase activity can be provided. A reagent kit can be also provided comprising at least one of MAPKAPK3, a polynucleotide encoding MAPKAPK3, a vector containing the polynucleotide and a transformant containing the vector; and at least one of TERT, a polynucleotide encoding TERT, a vector containing the polynucleotide and a transformant containing the vector.

According to the present invention, it can be carried out to inhibit the activation oftelomerase and telomerase activity. Therefore, according to the present invention, the prevention and/or treatment of a disease attributable to the enhanced telomerase activity can be carried out. Telomerase has a function to give immortality (unlimited lifespan) to a cell, of which activity has been detected in the immortalized cell such as a cancer cell. The inhibition oftelomerase acivation and the inhibition of telomerase activity according to the present invention enable to make the lifespan of a cancer cell limited and to make chromosome instable, thereby to induce cell death. Therefore, according to the present invention, for example, the prevention and/or treatment of a cancer disease such as breast cancer can be performed.

### BRIEF DESCRIPTION OF THE DRAWINGS

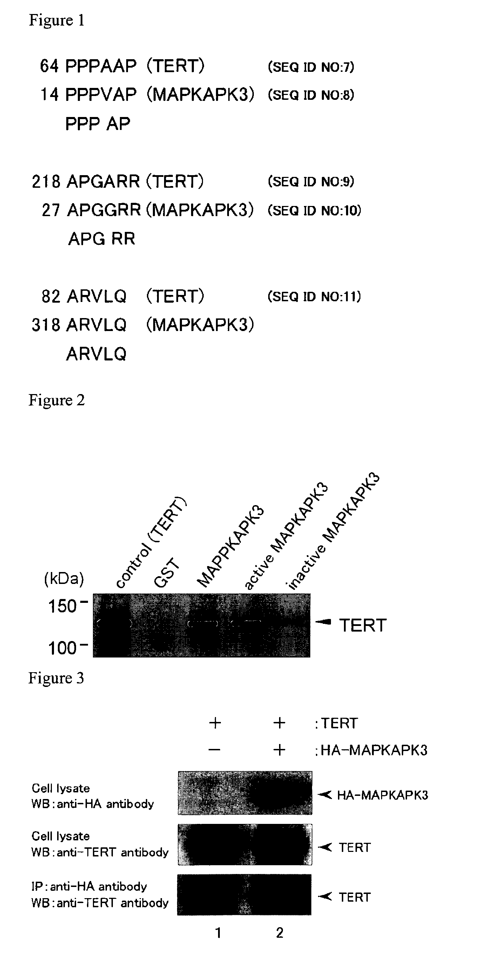

Fig. 1 shows the results of *in silico* prediction that MAPKAPK3 interacts with TERT that is a catalytic subunits oftelomerase. The region that exhibited a high score as a result of local alignment between TERT and MAPKAPK3 is shown. The amino acid sequences are represented in the single-letter code. The number in the figure indicates the position of the N-terminal amino acid of each region shown in the figure in the amino acid sequence of TERT or MAPKAPK3 (Example 1).
Fig. 2 shows the results that the binding of MAPKAPK3 or an active MAPKAPK3 to TERT that had been synthesized as ³⁵S-methonine labeled protein was detected by a pull-down method using glutathione sepharose. Moreover, an inactive MAPKAPK3 also binds to TERT, although the binding is weak comparing to MAPKAPK3 or active MAPKAPK3. GST has not bound to TERT. The arrow head indicates the position of TERT (Example 2).
Fig. 3 shows that co-precipitation of HA-MAPKAPK3 was observed (lowest panel) in a cell lysate of cells in which TERT was co-expressed with HA-MAPKAPK3 (lane 2), by immuno-precipitation (IP) with an anti-HA antibody followed by detecting proteins by Western blotting (WB) using anti-TERT antibody. The lane 1 shows the results concerning a cell lysate of cells into which the empty vector was transfected instead of the vector containing the polynucleotide encoding HA-MAPKAPK3 (Example 3).

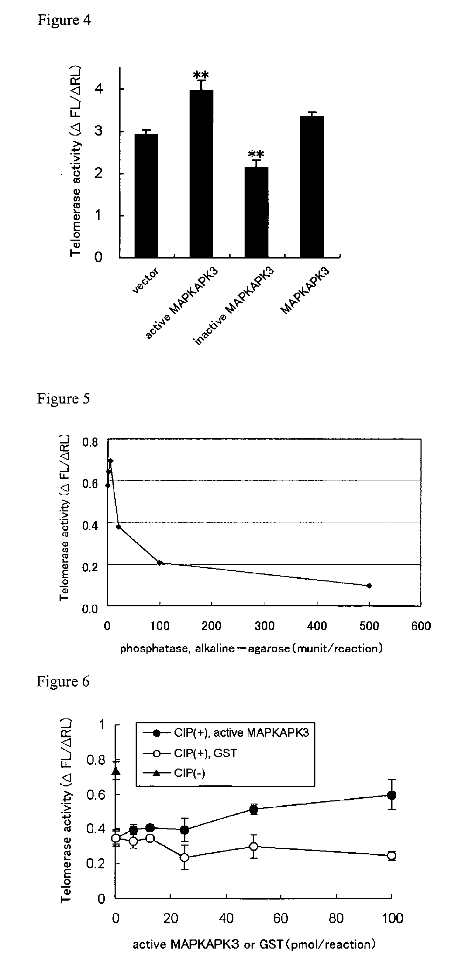

Fig. 4 shows the enhanced intracellular telomerase activity in cells in which active MAPKAPK3 was transiently expressed. On the other hand, that the intracellular telomerase activity was reduced in the cell in which inactive MAPKAPK3 was transiently expressed. The cell having telomerase activity was used in this experiment. In the graphical representation, the symbol "**" indicates that the significant difference (p<0.01) was observed by student t-test (Example 4).
Fig. 5 shows that telomerase activity was reduced by dephosphorylation treatment in a dose dependent manner of dephosphorylation enzyme (Example 5).
Fig. 6 shows that telomerase activity in a nuclear extract in which the telomerase activity had been reduced by dephosphorylation treatment, was enhanced in a dose dependent manner of the active MAPKAPK3, by adding an active MAPKAPK3 thereto to conduct phosphorylation reaction. The enhanced telomerase activity was not observed when phosphorylation reaction was conducted with adding GST. In the graphical representation, "CIP" means dephosphorylation enzyme (Example 5).

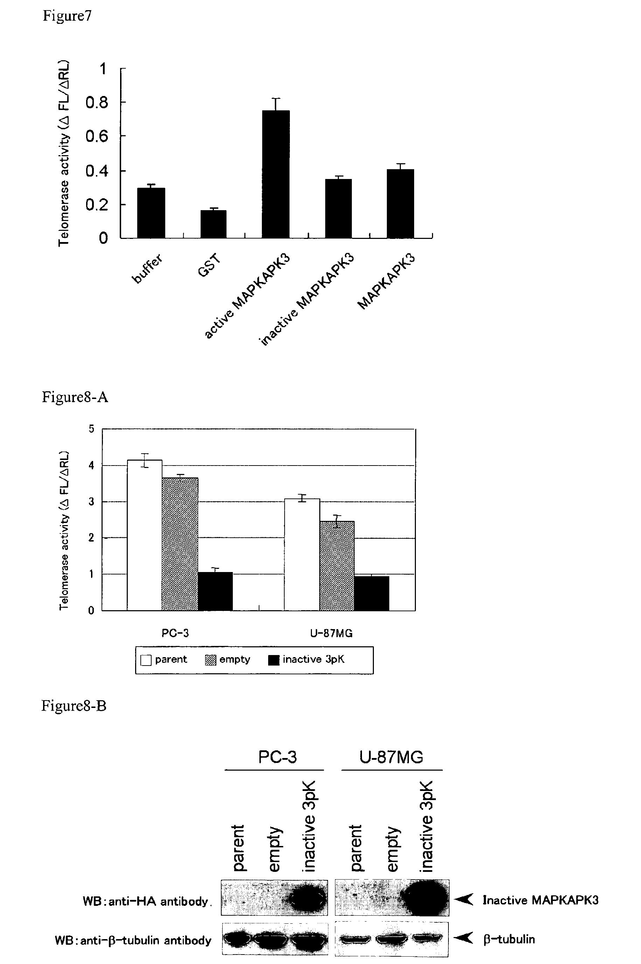

Fig. 7 shows that the telomerase activity in a nuclear extract in which the telomerase activity had been reduced by dephosphorylation treatment was enhanced by adding active MAPKAPK3 thereto to conduct phosphrylation reaction, but was not enhanced by MAPKAPK3 or inactive MAPKAPK3 (Example 5).
Fig. 8-A shows the significant reduction of telomerase activity in an inactive MAPKAPK3 expression retrovirus infected cell (inactive 3pK, in the graphical representation, shown by black column) in comparison with the parent line (in the graphical representation, shown by white column) or the empty virus infected cell (in the graphical representation, shown by oblique lines) (Example 6).
Fig. 8-B shows that inactive MAPKAPK3 was excessively expressed in an infected cell (inactive 3pK) that was a cancer cell line (PC-3 and U-87MG) infected with inactive MAPKAPK3 expression retrovirus. The expression amount of β-tubulin that was a control was almost the same in any one of the infected cell (inactive 3pK), the parent line and the empty virus infected cell. The detection of inactive MAPKAPK3 (with HA-tag at the N-terminal) was carried out by Western blot (WB) using an anti-HA antibody, and the detection of β-tubulin was carried out by Western blot (WB) using an anti-β-tubulin antibody (Example 6).

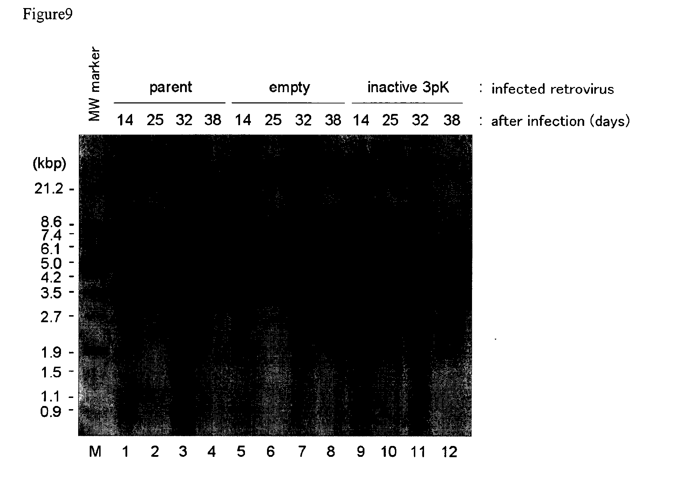

Fig. 9 shows that the shortened telomere length was observed in an infected cell (inactive 3pK) in a time dependent manner when a cancer cell line U-87MG was infected with an inactive MAPKAPK3 expression retrovirus. On the other hand, the change of telomere length was not observed in the parent line and the empty virus infected cell (Example 6).

### DETAILED DESCRIPTION OF THE INVENTION

### Embodiments of the present invention are explained in further detail below.

In the present specification, the term "polypeptide" may be used as a generic term which includes the followings: an isolated or a synthetic full-length protein; an isolated or a synthetic full-length polypeptide; and an isolated or a synthetic full-length oligopeptide. A protein, a polypeptide or an oligopeptide used herein comprises two or more amino acids that are bound to each other by peptide bond or modified peptide bond. Herein after, an amino acid may be represented by a single letter or by three letters.

In the present invention, the interaction of TERT, which is a catalytic subunit oftelomerase, with MAPKAPK3 was predicted *in-silico* according to the method described in the Patent Reference 1. Then, it has been demonstrated that TERT binds to MAPKAPK3. Furthermore, it has been clarified that telomerase activity is enhanced in a dose dependent manner of active MAPKAPK3. Moreover, it has been also demonstrated that TERT binds to inactive MAPKAPK3, and telomerase activity is reduced by the inactive MAPKAPK3 that binds to TERT, and as a result, telomere length of genomic DNA is shortened.

MAPKAPK3 is one of serine/threonine kinase, and has been reported that it is phosphorylated by a MAP kinase family (such as ERK, p38, JNK) to activate. As for the function of MAPKAPK3, it has been only reported that it is involved in luteinization mechanism. Moreover, it has been reported that MAPKAPK3 phosphorylates substrates such as HSP27, a transcription factor CREB and a transcription factor E47. However, there has been no report on the relationship between these substrates and telomerase.

There have been reports that p38, which is known to activate MAPKAPK3, is activated in a breast cancer tissue and non-small cell lung cancer tissue (Non-Patent References 7-9). Moreover, there have been reports that ERK, which is known to activate MAPKAPK3, is activated in a cancer tissue such as breast cancer, renal cell carcinoma, acute leukemia, glia cell carcinoma, prostatic cancer, neuroepithelial carcinoma, squamous cell carcinoma, liver cell carcinoma, prostatic cancer and the like (Non-Patent References 10-22). Therefore, it is believed that these cancer tissues may have a signal to induce telomerase activation through MAPKAPK3 activation.

The present invention has been clarified that MAPKAPK3 binds to TERT, and that active MAPKAPK3 enhances telomerase activity. From these findings, it is believed that MAPKAPK3 binds to TERT, and is subsequently phosphorylated by p38 or the like to become active MAPKAPK3, which then phosphorylates the TERT and results in the enhanced telomerase activity.

Therefore, it is believed that to inhibit the binding of MAPKAPK3 to TERT, or to inhibit the phosphorylation of TERT by active MAPKAPK3 can inhibit the activation of telomerase, and as a result, can inhibit the telomerase activity.

Furthermore, in the present invention, it has been clarified that TERT binds to an inactive MAPKAPK3, and that the inactive MAPKAPK3 capable of binding to TERT reduces the telomerase activity, resulting in the shortened telomere length. It is believed that an inactive MAPKAPK3 capable of binding to TERT inhibits telomerase activity by inhibiting the activation of telomerase and/or by inhibiting telomerase. The kinase activity of an inactive MAPKAPK3 capable of binding to TERT is reduced or deleted comparing to MAPKAPK3 and active MAPKAPK3. However, an inactive MAPKAPK3 capable of binding to TERT has the binding ability to TERT that is the substrate of MAPKAPK3. Therefore, it is highly conceivable that an inactive MAPKAPK3 capable of binding to TERT inhibits the activation of telomerase by competitively inhibiting the binding of TERT to MAPKAPK3, resulting in inhibiting the telomerase activity. Thus, telomerase activity can be inhibited by using an inactive MAPKAPK3 that binds to TERT.

Telomere is a simple repeated sequence located in the terminal portion of chromosome DNA in a eukaryotic cell, and contributes to maintaining the stability of chromosome. Therefore, when telomere length is shortened, the limited lifespan of a cancer cell and the cell death due to choromosome instablility are induced.

Since a cancer cell in which an inactive MAPKAPK3 capable of binding to TERT was expressed actually exhibited the reduced telomerase activity and the shortened telomere length, it is believed that the cell death can be induced by reducing telomerase activity using an inactive MAPKAPK3 capable of binding to TERT.

Telomerase is expressed in most of all the cancer cells regardless of kinds of cancers. Therefore, to use an inactive MAPKAPK3 capable of binding to TERT, to inhibit the binding of MAPKAPK3 to TERT, or to inhibit the phosphorylation of TERT by an active MAPKAPK3, enables to inhibit telomerase activity, and thereby enables to induce cell death of cancer cells, and as a result, enables to prevent and/or treat cancer diseases, such as breast cancer.

One embodiment of the present invention relates to a method for inhibiting telomerase activation comprising inhibiting the binding of MAPKAPK3 to TERT or inhibiting the phosphorylation of TERT by an active MAPKAPK3, and a method for inhibiting the telomerase activity using the method for inhibiting the activation of telomerase. Furthermore, one embodiment relates a method for inhibiting telomerase activity comprising using an inactive variant of MAPKAPK3, in which the variant is a variant capable of binding to TERT. The present invention relates to a method for inhibiting the telomerase activation and a method for inhibiting the telomerase activity preferably for human cancer tissues, such as the tissues of breast cancer, renal cell carcinoma, acute leukemia, glia cell carcinoma, prostatic cancer neuroepithelial carcinoma, squamous cell carcinoma, liver cell carcinoma, prostatic cancer, and non-small cell lung cancer, more preferably for a human breast cancer tissue.

The term "active MAPKAPK3" refers to MAPKAPK3 that has been activated so that to exhibit kinase activity. An active MAPKAPK3 can be MAPKAPK3 that has been phosphorylated and activated by MAP kinase family (ERK, p38, JNK and the like) and the like. It may be preferably MAPKAPK3 that is activated by phosphorylation of its threonine residues at positions 201 and 313 in the amino acid sequence of MAPKAPK3 by MAP kinase family and the like, so as to exhibit higher acitivty comparing to MAPKAPK3 before being subjected to phosphorylation. Moreover, it may be a MAPKAPK3 variant into which a mutation is introduced so as to exhibit kinase activity in nature or using genetic engineering method. Such a MAPKAPK3 variant is exemplified by a variant (SEQ. ID.No.8) in which the threonine residues at positions 201 and 313 of the amino acid sequence of MAPKAPK3 have been substituted into glutamic acid.

The term "MAPKAPK3" refers to a protein consisting of the amino acid sequence (SEQ. ID.No.2) encoded by the polynucleotide shown by the nucleotide sequence set forth in SEQ ID NO: 1 in the sequence listing, which is not activated by phosphorylation or the like, and exhibits no kinase activity or weak kinase activity.

TERT gene and a protein encoded by the gene are a gene and protein which are shown by the sequences set forth in SEQ ID NO: 3 and SEQ ID NO: 4 in the sequence listing, respectively.

The term "the binding of MAPKAPK3 to TERT" refers to the interaction of MAPKAPK3 with TERT so as to form a complex by non-covalent bond such as hydrogen bond, hydrophobic bond, electrically static interaction or the like. The binding of MAPKAPK3 to TERT only at the portion of these molecules is enough to be referred as "the binding" mentioned herein. For example, an amino acid which is not involved in the binding of MAPKAPK3 to TERT may be contained in the amino acid that constitutes MAPKAPK3 or TERT.

The binding of MAPKAPK3 to TERT can be detected by a method well known in the art, such as an immuno-precipitation method for confirming co-precipitated product, a two-hybrid method, a Western blotting, a fluorescence resonance energy transfer method and the like, or by using these methods in combination.

For example, first, MAPKAPK3 to which glutathione S transferase (GST) has been fused at N-terminal (GST-MAPKAPK3) is brought into a reaction with TERT synthesized as ³⁵S-methionine labeled protein. Subsequently GST-MAPKAPK3 is collected by glutathione sepharose and developed by sodium dodecyl sulfate polyacrylamide gel electrophoresis (SDS-PAGE). The binding of MAPKAPK3 to TERT can be detected by detecting TERT by an autoradiography (see Example 2).

Alternatively, after reacting the N -terminal HA-tagged MAPKAPK3 with TERT, the MAPKAPK3 is subjected to immunoprecipitation using an anti-HA antibody. The binding of MAPKAPK3 to TERT can be detected by performing Western blotting against the co-precipitated product using an anti-TERT antibody (see Example 3).

The term "telomerase activation" refers to the fact that telomerase activity is enhanced as a result of phosphorylation of TERT that comprises telomerase by an active MAPKAPK3 comparing to the telomerase activity before it is subjected to phosphorylation

MAPKAPK3, TERT and their genes are not limited to the protein and genes shown by the sequences as descrived above, and can be a protein and polynucleotide having one to several mutations in the above-described sequence as long as these have the functions of MAPKAPK3 and TERT which are known in general. Moreover, for the desired purpose to enhance or delete the functions of these proteins and genes, the variant in which one to several mutations has been introduced in the above-described sequences can be also utilized.

MAPKAPK3 gene and TERT gene can be easily obtained from a suitable origin (for example, cell human breast cancer) in which expressions of the respective genes are observed, by using a cloning method known by itself and the like. A gene encoding an active MAPKAPK3 can be obtained as a MAPKAPK3 mutant gene by introducing a mutation into MAPKAPK3 gene, using a well known method for introducing a mutation, such as a site specific mutation method or the like. The proteins encoded by these genes can be obtained, for example, by a well known genetic engineering method using the respective genes.

The inhibition of the binding of MAPKAPK3 to TERT can be carried out by utilizing a compound that inhibits the binding of MAPKAPK3 to TERT. As used herein, a compound having such an inhibitory effect (as the example described later, polypeptides having a competitive inhibiting effect, antibody and compound having a lower molecular weight and the like are listed) is referred to as an inhibitor.

A compound that inhibits the binding of MAPKAPK3 to TERT is preferably a compound that specifically inhibits the binding, and more preferably, a compound with a lower molecular weight which specifically inhibits the binding. "Specifically inhibit the binding of MAPKAPK3 to TERT" denotes that inhibit the binding strongly, but does not inhibit or weakly inhibit the binding between the other proteins".

As a compound that inhibits the binding of MAPKAPK3 to TERT besides the above-described compounds, a polypeptide comprising the amino acid sequence of a site where MAPKAPK3 binds to TERT can be exemplified. Such a polypeptide can competitively inhibit the protein-protein binding. Such a polypeptide can be obtained by designing polypeptides based on the amino acid sequence of MAPKAPK3 or TERT, synthesizing them by peptide synthesis methods well-known in the art, and selecting a polypeptide that inhibits the binding of MAPKAPK3 to TERT from them. A polypeptide having an amino acid sequence derived from the thus specified polypeptide, in which a mutation such as a deletion, substitution, addition or insertion of one to several amino acids was introduced, is also included in the scope of the present invention. As for the polypeptide into which such a mutation has been introduced, a polypeptide inhibiting the binding of MAPKAPK3 to TERT is preferably used. A polypeptide having the mutation may be a naturally existing polypeptide or a polypeptide in which a mutation was introduced. Techniques for introducing a mutation such as a deletion, substitution, addition or insertion are known. For example, the Ulmer technique (Non-patent Reference 23) may be utilized. When introducing a mutation as described above, in view of avoiding a change in the fundamental properties (such as physical properties, function, physiological activity, and immunological activity) of the polypeptide, mutual substitution among homologous amino acids (polar amino acids, non-polar amino acids, hydrophobic amino acids, hydrophilic amino acids, positively-charged amino acids, negatively-charged amino acids and aromatic amino acids or the like) may be readily conceived. Furthermore, these usable polypeptides can be modified to the extent that no significant functional change is involved, such as modification of its constituent amino group or carboxyl group and the like by an amidation and the like.

The aforementioned polypeptide can be produced by common methods that are known in peptide chemistry. A method described in the References (Non-patent documents 24 and 25), for example, may be used, although the methods are not limited thereto, and known methods can be broadly utilized. Particularly, a peptide synthesis method using an ordinary liquid phase method and solid phase method, such as, for example, the Fmoc method, can be exemplified. Moreover, an amino acid synthesizer that is commercially available can be used for producing the polypeptide. Alternatively, a gene engineering technology can be also used for producing the polypeptide. For example, a gene encoding a polypeptide of interest can be used to prepare a recombinant expression vector that can express the gene in a host cell, followed by transfection of the recombinant expression vector into a suitable host cell such as E. coli to obtain a transformant. Culturing the transformant and collecting the polypeptide of interest from the obtained culture product achieves the production of the polypeptide.

The inhibition of the binding of MAPKAPK3 to TERT can be also carried out by using an antibody that recognizes MAPKAPK3 or TERT, and inhibits the binding of MAPKAPK3 to TERT. Such an antibody can be produced by known methods for preparing an antibody, using MAPKAPK3 or TERT itself, or a fragment thereof, preferably a polypeptide comprising the amino acid sequence of a site where MAPKAPK3 binds to TERT as antigen.

The term "phosphorylation of TERT by an active MAPKAPK3" refers to the fact that γ-phosphorylate group of adenosin tri-phosphate (ATP) is transferred to hydroxyl group of serine/threonine residues within the amino acid constituting TERT by an active MAPKAPK3. In general, the enzyme activity of a protein having an enzyme activity is often regulated by phosphorylation. Therefore, it is believed that the reverse transcription enzyme activity of TERT is promoted by phosphorylation, which results in the enhanced telomerase activity.

The detection of phosphorylation of TERT by an active MAPKAPK3 and the detection of phosphorylated TERT can be carried out by a method known in the art. It is believed that in vivo, MAPKAPK3 binds to TERT followed by the activation of the MAPKAPK3 by MAP kinase family and the like such as ERK, JNK, p38. However, as shown in examples, an active MAPKAPK3 variant has bound to TERT and has enhanced telomerase activity in a dose dependent manner of the active MAPKAPK3 variant. Therefore, the phosphorylation of TERT by an active MAPKAPK3 can be carried out by making the active MAPKAPK3 co-exist with TERT. An active MAPKAPK3 can be preferably exemplified by a MAPKAPK3 variant, in which the threonine residues at positions 201 and 313 of amino acid sequence of MAPKAPK3 are substituted with glutamic acid.

A method for detecting phosphorylation of TERT by an active MAPKAPK3 and phosphorylated TERT can be exemplified concretely by the following methods.

The phosphorylated TERT by an active MAPKAPK3 can be detected by contacting the active MAPKAPK3 with TERT in an kinase buffer containing a radioactive isotope labeled ATP (for example, [γ-³²P]ATP) for reaction, subsequently developing the reaction product by SDS-PAGE and then measuring the radio activity by an autoradiography.

Alternatively, the phosphorylated TERT by an active MAPKAPK3 can be detected by immobilizing TERT onto a well of a micro-titer plate by a known method in the art, adding ATP and an active MAPKAPK3 to the well to contact the TERT with active MAPKAPK3 for reaction, and then using an antibody recognizing phosphorylated TERT which has been previously labeled with Europium (Eu) to conduct a reaction of phosphorylated TERT with the antibody, subsequently detecting a time-resolved fluorescence.

The phosphorylated TERT by an active MAPKAPK3 can be detected by contacting an active MAPKAPK3 with a donor bead to which TERT has been bound for reaction, adding to the reaction solution with an acceptor bead to which an anti-phosphorylated TERT antibody has been bound, and then detecting a signal that is generated only when both beads are to be in close proximity (for example, light having a certain wavelength generated as a result of the activation of a fluorescent substance contained in the acceptor bead, by singlet state oxygen generated when the donor bead was laser-irradiated).

Alternatively, the phosphorylated TERT by an active MAPKAPK3 can be detected by using scintillation proximity assay (SPA) well known in the art.

The method exemplified above can be also carried out using a partial polypeptide of TERT which is subjected to phosphorylation by an active MAPKAPK3 as a substrate instead of TERT. From the report on the site where HSP27 and eEF2 kinase, which are the substrate of MAPKAPK3, are phosphorylated by MAPKAPK3, it is estimated that the site where TERT is phosphorylated by an active MAPKAPK3 is the site corresponding to a partial polypeptide consisting of four amino acid of RXXS (as used herein, X can be any of amino acid). Among the partial polypeptides of TERT containing such a site, the partial polypeptide of TERT which has been proved to be phosphorylated by an active MAPKAPK3 can be used as a substrate instead of TERT. The partial polypeptide of TERT can be synthesized by a method well known in the art.

The inhibition of phosphorylation of TERT by an active MAPKAPK3 can be carried out by utilizing a compound that inhibits the phosphorylation of TERT by an active MAPKAPK3. As a compound that inhibits the phosphorylation of TERT by an active MAPKAPK3, for example, a compound that inhibits the binding of an active MAPKAPK3 to TERT. A compound for inhibiting the binding of an active MAPKAPK3 to TERT can inhibit the interaction of an active MAPKAPK3 with TERT, therefore, it is believed that such a compound inhibits the phosphorylation of TERT by an active MAPKAPK3. Moreover, it is believed that MAPKAPK3 binds to TERT, and is subsequently phosphorylated by ERK, JNK, and/or p38 or the like to become active MAPKAPK3, which then phosphorylates the TERT and results in the enhanced telomerase activity, therefore it is believed that a compound for inhibiting the binding of MAPKAPK3 to TERT inhibits the interaction between MAPKAPK3 with TERT, and thereby inhibits the generation of an active MAPKAPK3 and the phosphorylation of TERT by an active MAPKAPK3 which occurs following to the generation. Therefore, a compound that inhibits the binding of MAPKAPK3 to TERT can be preferably exemplified as a compound that inhibits the phosphorylation of TERT by an active MAPKAPK3. In addition, as a compound that inhibits the phosphorylation of TERT by an active MAPKAPK3, a compound that inhibits the kinase activity of an active MAPKAPK3 is exemplified.

Alternatively, the phosphorylation of TERT by an active MAPKAPK3 can be inhibited by reducing the expression of MAPKAPK3 in cells, such as cancer cells, by means ofRNA interference to reduce the generatin of an active MAPKAPK3. As a result, telomerase activity can be reduced or deleted. Therefore, siRNA (small interfering RNA) which reduces or deletes telomerase activity by such a means of RNA interference can be also exemplified as a compound that inhibits telomerase activity. Introduction of siRNA into a cancer cell induces the reduced telomerase activity in the cell, thereby makes the telomere length at chromosome end shorten with every cell division, and can induce cell death.

The term "inactive variant of MAPKAPK3" refers to a MAPKAPK3 into which a mutation such as deletion, substitution, addition, insertion or the like of amino acid has been introduced, and has a reduced or deleted kinase activity comparing to MAPKAPK3 and an active MAPKAPK3. An inactive variant of MAPKAPK3 may be a naturally existing one, or may be a variant into which a mutation has been artificially introduced. Techniques for introducing such a mutation are well known in the art, and can be conducted using, for example, the Ulmer technique (Non-patent Reference 23). An inactive variant of MAPKAPK3 may be preferably MAPKAPK3 into which a mutation has been introduced into at least any one of the site selected from the sites, such as the phosphorylation site of MAPKAPK3 by the kinase for phosphorylating and activating MAPKAPK3 such as ERK, JNK and/or p38 or the like, the binding site of MAPKAPK3 and TERT and the binding site of MAPKAPK3 and ATP. As a phosphorylation site of MAPKAPK3 by the kinase for phosphorylating and activating MAPKAPK3, the amino acid residues at positions 201 and 313 of the amino acid sequence of MAPKAPK3 has been reported (Non-Patent Reference 26). As a binding site of MAPKAPK3 and ATP, the amino acid residue at position 73 of the amino acid sequence of MAPKAPK3 has been reported (Non-Patent Reference 26). Therefore, as an inactive variant of MAPKAPK3, an inactive variant of MAPKAPK3 with a mutation introduced into an amino acid residue at least one of the positions 73, 201 and 313 of amino acid sequence of MAPKAPK3 can be exemplified.

An inactive variant is more preferably exemplified by an inactive variant of MAPKAPK3 that binds to TERT. Such an inactive variant is exemplified by the protein shown by the amino acid sequence set forth in SEQ ID No.6 in the sequence listing. The inactive variant is an inactive variant of MAPKAPK3 in which threonine residues at positions 201 and 313 of the amino acid sequence of MAPKAPK3 has been substituted with alanine. The inactive variant bound to TERT (see Example 2 and Fig. 2), and reduced the intracellular telomerase activity (see Example 4 and Fig. 4) when it was expressed in a cell having telomerase activity. Moreover, the inactive variant reduced the intracellular telomerase activity when it was expressed in a cancer cell having telomerase activity, and thereby made the telomere length be shortened (see Example 6, Fig. 8-A, Fig. 8-B and Fig. 9). From these findings, it is believed that the inactive variant inhibits telomerase activation and/or telomerase in a cell, and thereby inhibits the telomerase activity.

An inactive variant is more preferably exemplified by an inactive variant of MAPKAPK3 that binds to TERT and competitively inhibits the binding of MAPKAPK3 to TERT. Such an inactive variant inhibits the telomerase activation by inhibiting the MAPKAPK3 to TERT and thereby can inhibit the telomerase activity. The protein shown by the amino acid sequence set forth in SEQ ID No.6 in the sequence listing bound to TERT, and reduced the intracellular telomerase activity when it was expressed in a cell having telomerase activity or in a cancer cell. Namely, it is believed that the protein has the dominant negative effect against MAPKAPK3. Therefore, the present inventors believe that the protein is an inactive variant of MAPKAPK3 that competitively inhibits the binding of MAPKAPK3 to TERT.

The binding of an inactive MAPKAPK3 to TERT can be detected by a method similar to a method for detecting the binding of the foregoing MAPKAPK3 to TERT.

Another embodiment of the present invention relates to a method of identifying a compound that inhibits the binding of MAPKAPK3 to TERT. The present method of identifying a compound can be carried out by utilizing a pharmaceutical screening system known in the art. For example, it can be carried out using a method for detecting the binding of MAPKAPK3 to TERT as described above.

A method of identifying a compound that inhibits the binding of MAPKAPK3 to TERT can be carried out concretely by selecting the conditions that allow for the interaction of a compound to be tested (hereinafter, referred to the test compound) with MAPKAPK3 and/or TERT, contacting the test compound with MAPKAPK3 and/or TERT under the condition, employing a system that uses a signal and/or a marker capable of detecting the binding of MAPKAPK3 to TERT, and then detecting the presence, the absence or the change of the signal and/or the marker. For example, in the case that the signal generated by the binding of MAPKAPK3 to TERT or the marker of the binding shows a change, such as reduction or disappearance, when contacting a test compound with MAPKAPK3 or TERT, it can be determined that the test compound inhibits the binding of MAPKAPK3 to TERT. In such an identification method, the test compound may be previously contacted with MAPKAPK3 and/or TERT, and then the binding reaction of MAPKAPK3 to TERT may be conducted. Alternatively, the test compound may be allowed to co-exist in the binding reaction of these proteins. The conditions that allow for the interaction of a test compound with MAPKAPK3 and/or TERT may be a condition in vitro or in vivo. For example, a cell in which MAPKAPK3 is coexpressed with TERT may be used. Such a cell can be obtained by transfecting a cell using a suitable vector containing a polynucleotide encoding MAPKAPK3 and a suitable vector containing a polynucleotide encoding TERT by means of a conventional genetic engineering method. Alternatively, such a cell can be also obtained by transfecting a cell in which the telomerase activity has been observed, using a polynucleotide encoding MAPKAPK3. Furthermore, a similar identification method can be constructed using an active MAPKAPK3, since an active MAPKAPK3 bound to TERT, as indicated in Example 2. In this case, the MAPKAPK3 may be activated and phosphorylated in cells by coexpressing of a polynucleotide encoding ERK, JNK and/or p38. As used herein, the term "signal" refers to a substance that can be detected itself directly based on its physical properties or chemical properties. The term "marker" refers to a substance that can be indirectly detected based on its physical properties or biological as an index. A signal can be exemplified by known ones, such as luciferase, a radioactive isotope and the like. A marker can be exemplified by known ones, such as a reporter gene (for example chloramphenicol acetyl transferase gene and the like), and epitope tags for detection (for example, 6 x His-tag and the like). A method for detecting these signals or markers is well known to those skilled in the art. The binding of MAPKAPK3 to TERT can be easily detected by detecting the presence or absence of these proteins and/or by measuring the change of the amount thereof. The detection of the binding of these proteins can be carried out by a well known method for detecting a protein or peptide, for example Western blotting method and the like.

A method of identifying a compound that inhibits the binding of MAPKAPK3 to TERT can be carried out concretely, for example, using a in vitro binding assay system generally used and known in the art, which comprises immobilizing either of MAPKAPK3 or TERT onto a solid-phase, conducting the binding reaction using the other one that is labeled with the signal, and measuring the labeled signal quantitatively. In the case that the labeled signal shows reduction or disappearance under co-existence of the test compound in such an assay system as compared with the signal in the absence of the compound, it can be determined that the test compound inhibits the binding of MAPKAPK3 to TERT.

A method of identifying a compound that inhibits the binding of MAPKAPK3 to TERT can be carried out by reacting GST fusion MAPKAPK3 or GST fusion active MAPKAPK3 with TERT in vitro, and using an assay system (see Example 2) for detecting the binding of both proteins by a pull-down method using GST. The detection of the binding of MAPKAPK3 to TERT in such an assay system can be carried out by detecting a labeling substance that has previously been labeled to TERT. Any labeling substances generally used for detecting a protein can be used in this assay. Concretely, a radioactive isotope such as ³⁵S or the like can be preferably exemplified. In the case that the amount of a labeling substance detected under co-existence of the test compound in such an assay system is reduced or deleted comparing to the amount of the labeling substance detected in the absence of the test compound, it can be determined that the test compound inhibits the binding of MAPKAPK3 to TERT.

Alternatively, a method of identifying a compound that inhibits the binding of MAPKAPK3 to TERT can be carried out using an assay system (see Example 3) for detecting the intracellular binding reaction using a cell obtained by transfecting a polynucleotide encoding TERT and a polynucleotide encoding MAPKAPK3 into an animal cell. The detection of the binding of MAPKAPK3 to TERT in such an assay system can be carried out by a known method such as an immunoprecipitation method, Western blotting and the like. In the case that the amount of binding protein is reduced or the binding is not detected under co-existence of the test compound in such an assay system, comparing to the results obtained in the absence of the test compound, it can be determined that the test compound inhibits the binding of MAPKAPK3 to TERT

Alternatively, a method of identifying a compound that inhibits the binding of MAPKAPK3 to TERT can be also carried out by utilizing a well known two-hybrid method. For example, it can be achieved by using a yeast, a eukaryotic cell or the like into which a plasmid for expressing a fusion protein of MAPKAPK3 and DNA binding protein, a plasmid for expressing a fusion protein of TERT and transcription activating protein and a plasmid containing a reporter gene such as lacZ or the like that is connected to a suitable promoter gene are introduced, and comparing an expression amount of reporter gene under co-existence of the test compound to an expression amount of reporter gene in the absence of the test compound. The expression amount of reporter gene under co-existence of a test compound was reduced comparing to the expression amount of reporter gene in the absence of a test compound, it can be determined that the test compound has the activity for inhibiting the binding of MAPKAPK3 to TERT.

A method of identifying a compound that inhibits the binding of MAPKAPK3 to TERT can be also carried out by utilizing a surface plasmon resonance sensor such as BIACORE system or the like, an SPA method, or a method that employing fluorescence resonance energy transfer (FRET).

Another embodiment of the present invention relates to a method of identifying a compound that inhibits the phosphorylation of TERT by an active MAPKAPK3. The method of identifying a compound can be carried out by utilizing a pharmaceutical screening system that is well known in the art. For example, it can be carried out by utilizing a method for detecting the phosphorylation of TERT by an active MAPKAPK3 described above.

A method of identifying a compound that inhibits the phosphorylation of TERT by an active MAPKAPK3 can be carried out concretely by selecting the conditions that allow for the interaction of a test compound with an active MAPKAPK3 and/or TERT, contacting the test compound with the active MAPKAPK3 and/or TERT under the condition, employing a system that uses a signal and/or a marker capable of detecting the phosphorylation of TERT by the active MAPKAPK3, and then detecting the presence, the absence or the change of the signal and/or the marker. For example, in the case that the signal generated by the phosphorylation of TERT by the active MAPKAPK3 or the marker of the binding shows a change, such as reduction or disappearance, when contacting a test compound with MAPKAPK3 and/or TERT, it can be determined that the test compound inhibits the phosphorylation of TERT by the active MAPKAPK3. The conditions that allow for the interaction of a test compound with MAPKAPK3 and/or TERT may be a condition in vitro or in vivo. For example, a cell in which MAPKAPK3 is coexpressed with TERT may be used.

A method of identifying a compound that inhibits the phosphorylation of TERT by an active MAPKAPK3 can be carried out concretely, for example, using a nuclear extract prepared from the cells in which the telomerase activity is observed, by conducting dephosphorylation treatment of nuclear extract, and subsequently utilizing an assay system (see Example 5) to measure the enhanced telomerase activity by adding an active MAPKAPK3. In the case that the enhanced telomerase activity was inhibited under co-existence with a test compound in such an assay system, it can be determined that the test compound is a compound that hinhibits the phosphorylation of TERT. Alternatively, the identification of a compound that inhibits the phosphorylation of TERT can be carried out using such an assay system by detecting the phosphorylation of TERT instead of measuring the enhanced telomerase.

A method of identifying a compound that inhibits the phosphorylation of TERT by an active MAPKAPK3 can be also carried out using an assay system in which the aforementioned method for carrying out the phosphorylation of TERT by an active MAPKAPK3 and detection of the phosphorylated TERT is utilized. In the case that the phosphorylation of TERT by the test compound under co-existence of the test compound in such a system is reduced or disappeared comparing to the phosphorylation in the absence of the test compound, it can be determined that the test compound inhibits the phosphorylation of TERT by the active MAPKAPK3.

Proteins such as MAPKAPK3, active MAPKAPK3, inactive MAPKAPK3 and TERT can be those prepared from cells in which these are expressed by means of genetic engineering techniques, or from any biological samples, or can be the products of cell-free synthesis systems or the chemical synthesis products. Also, these can be those which are subsequently further purified. Further, these can be those which are expressed in a cell containing at least one gene of the gene encoding the each protein. The cell can be a transfectant obtained by transfecting a vector that contains a gene encoding the protein. Moreover, these can be labeled by ligating a different type of protein or polypeptide (GST, β-galactosidase, an immunoglobulin Fc fragment (such as IgG), a tag peptide (such as His-tag, Myc-tag, HA-tag, FLAG-tag, or Xpress-tag)) thereto at the N-terminus or the C-terminus, directly or indirectly via a linker peptide and the like, by means of, for example, genetic engineering techniques, as long as it has no influence upon the binding of MAPKAPK3 to TERT, and upon the function thereof, such as phosphorylation of TERT by active MAPKAPK3.

Examples of a test compound include a compound derived from a chemical library or natural products, as well as a compound obtained by drug design based on the primary structure or tertiary structure of active MAPKAPK3 or TERT. Alternatively, a compound obtained by drug design based on the structure of a polypeptide that comprises an amino acid sequence of a binding site of MAPKAPK3 to TERT is also suitable as a test compound.

The other Embodiment of the present invention relates to a telomerase activation inhibitor and a telomerase activity inhibitor, comprising inhibiting the binding of MAPKAPK3 to TERT. The telomerase activation inhibitor and telomerase activity inhibitor are preferably exemplified by a telomerase activation inhibitor and a telomerase activity inhibitor each of which comprises at least one compound that inhibits the binding of MAPKAPK3 to TERT, such as the aforementioned compound.

The other Embodiment of the present invention relates to a telomerase activation inhibitor and a telomerase activity inhibitor, comprising inhibiting the phosphorylation of TERT by an active MAPKAPK3. The telomerase activation inhibitor and telomerase activity inhibitor are preferably exemplified by a telomerase activation inhibitor and a telomerase activity inhibitor each of which comprises at least one compound that inhibits the phosphorylation of TERT by an active MAPKAPK3, such as the aforementioned compound. More preferably, a telomerase activity inhibitor containing the aforementioned inactive variant of MAPKAPK3 or a telomerase activation inhibitor containing the inactive variant can be exemplified.

The determination of the telomerase activation inhibitory action and the telomerase activity inhibitory action can be carried out, for example, by a method such as TRAP method (Non-Patent Reference 27), direct telomerase assay (Non-Patent Reference 28), stretch PCR assay (Non-Patent Reference 29), hybridization protection assay (Non-Patent Reference 30), TRE assay (Non-Patent Reference 31), rapid direct telomerase assay (Non-Patent Reference 32) and the like. Alternatively, the determination of these actions can be carried out according to a method that has been reported by Padison et al (Non-Patent Reference 33) and a method that is described concretely in the examples of the present specification.

The other Embodiment of the present invention relates to a pharmaceutical composition containing the above-described compound and the above-described inhibitor. The above-described compound and the above-described inhibitor can be selected in consideration of a balance between the biological usefulness and toxicity to prepare a pharmaceutical composition. In preparation of the pharmaceutical composition, these compounds and inhibitors may be used alone or in combination. The pharmaceutical composition according to the present invention can be utilized for an agent for preventing and/or treating a disease attributable to the telomerase action and the enhanced activity of the telomerase as well as for a method for preventing and/or treating the disease. Examples of the disease attributable to the enhanced telomerase activity include cancer diseases.

The telomerase activity has been observed in a large number of tumors regardless of the kinds of tumors. Thus, it is believed that the pharmaceutical composition according to the present invention is effective for inhibition of proliferation of a variety of cancer cells. Therefore, in the case where the present pharmaceutical composition is used for a cancer disease, the kind of a tumor to be targeted is not particularly limited, and it can be applied to either solid tumor or non-solid tumor. The kind of a solid tumor or non-solid tumor is also not particularly limited, and the present pharmaceutical composition can be applied to all kinds of tumors having telomerase activity. A cancer disease is exemplified by a solid tumor such as a stomach cancer, esophageal cancer, colon cancer, small intestinal cancer, duodenal carcinoma, lung cancer, liver cancer, gallbladder cancer, pancreatic cancer, renal cancer, bladder cancer, oral cancer, osteocarcinoma, skin cancer, breast cancer, uterine cancer, prostate cancer, brain tumor, neuroblastoma and the like, or a non-solid tumor such as leukemia, malignant lymphoma, and the like. However, the application target of the present pharmaceutical composition is not limited to these diseases. More preferably, the present pharmaceutical composition can be further applied to a cancer disease in which the activation of MAPKAPK3 and the enhanced expression of its gene are observed, or a cancer disease in which the activation of a protein involving in the activation of MAPKAPK3 is observed. It has been reported that p38 known to activate MAPKAPK3 is activated in a breast cancer tissue and a non-small lung cancer tissue. Furthermore, it has been reported that ERK known to activate MAPKAPK3 is activated in a cancer tissue such as breast cancer, renal cell carcinoma, acute leukemia, glia cell tumor, prostate cancer, neuroepithelioma, squamous carcinoma, liver cell carcinoma and the like. Therefore, the present pharmaceutical composition is preferably applied to breast cancer, renal cell carcinoma, acute leukemia, glia cell tumore, prostate cancer, neuroepithelioma, squamous carcinoma, liver cell carcinoma, prostate cancer and non-small lung cell cancer, and is more preferably applied to breast cancer.

The agent for preventing and/or treating the diseases according to the present invention can be a pharmaceutical agent prepared by using at least any one of the members consisting of the aforementioned compounds and inhibitor so as to contain the effective amount thereof. Generally, the pharmaceutical agent is preferably prepared as a pharmaceutical composition that includes one or more kinds of a pharmaceutical carrier.

An amount of the effective ingredient containing in the pharmaceutical preparation can be suitably selected from wide range. In general, a suitable amount may fall within a range of approximately 0.00001 to 70 wt%, preferably approximately 0.0001 to 5 wt%.

Examples of a pharmaceutical carrier may include a filler, extender, binder, wetting agent, disintegrator, lubricant, diluent and excipient that are generally used in accordance with the form of use of the formulation. These can be suitably selected and used in accordance with the administration form of the preparation to be obtained.

More concretely, the pharmaceutical carrier is exemplified by water, a pharmaceutically acceptable organic solvent, collagen, polyvinyl alcohol, polyvinylpyrrolidone, carboxyvinyl polymer, sodium alginate, soluble dextran, sodium carboxymethyl starch, pectin, xanthan gum, acacia gum, casein, gelatin, agar, glycerin, propylene glycol, polyethylene glycol, vaseline, paraffin, stearyl alcohol, stearic acid, human serum albumin, mannitol, sorbitol and lactose. One or a combination of two or more kinds of these may be suitably selected and used in accordance with the dosage form according to the present invention.

As desired, various components that may be used in conventional protein preparation, such as a stabilizer, bacteriocide, buffer agent, isotonizing agent, chelating agent, pH adjuster, or surfactant, may be suitably used for preparation.

Examples of a stabilizer may include human serum albumin, common L-amino acids, sugars and cellulose derivatives. These can be used independently or in combination with a surfactant and the like. Especially, use of these in such a combination may give increased stability of an effective ingredient. The aforementioned L-amino acid is not particularly limited, and may be any one of glycine, cysteine, glutamic acid and the like. A sugar is not particularly limited, and may be any one of monosaccharides (such as glucose, mannose, galactose and fructose), sugar alcohols (such as mannitol, inositol and xylitol), disaccharides (such as sucrose, maltose and lactose), polysaccharides (dextran, hydroxypropylstarch, chondroitin sulfate and hyaluronic acid), derivatives thereof and so on. A cellulose derivative is not particularly limited, and may be any one of methylcellulose, ethylcellulose, hydroxyethylcellulose, hydroxypropylcellulose, hydroxypropylmethlcellulose, sodium carboxymethylcellulose and the like. A surfactant is not particularly limited, and can be both an ionic surfactant and a non-ionic surfactant. Examples of a surfactant may include polyoxyethyleneglycol sorbitan alkyl ester system, polyoxyethylene alkyl ether system, sorbitan monoacyl ester system, fatty acid glyceride system.

Examples of a buffer agent may include boric acid, phosphoric acid, acetic acid, citric acid, ε-aminocaproic acid, glutamic acid and/or a salt thereof (for example, an alkaline metal salt and an alkali earth metal salt, such as sodium salt, kalium salt, calcium salt and magnesium salt).

Examples of an isotonizing agent may include sodium chloride, kalium chloride, sugars and glycerin.

Examples of a chelating agent may include sodium edentate and citric acid.

The pharmaceutical agent and pharmaceutical composition according to the present invention can be used as a solution preparation. Alternatively, they can be freeze-dried so as to be in good state in preservation and can be used by dissolving them in water, a buffered solution containing saline and the like, and so on and then adjusting to suitable concentration at the time of using.

Suitable dosage ranges of the pharmaceutical composition are not particularly limited, and can be determined according to the following: effectiveness of the ingredients contained therein; the administration form; the route of administration; the type of disease; the characteristics of the subject (e.g., body weight, age, symptomatic conditions and whether being taking other pharmaceutical agent) and the judgment of the physician in charge. In general, a suitable dosage may fall, for example, within a range of about 0.01 *µ*g to 100 mg per 1 kg of the body weight of the subject, and preferably within a range of about 0.1 µg to 1 mg per 1 kg. However, a dosage may be altered using conventional experiments for optimization of a dosage that are well known in the art. The aforementioned dosage can be divided for administration once to several times a day. Alternatively, periodic administration once every few days or few weeks can be employed.

When administering the pharmaceutical composition of the present invention, the pharmaceutical composition may be used alone or may be used together with other compounds or pharmaceutical agents necessary for the treatment. For example, combination with the active ingredient of the other telomerase inhibitor or of the anti-tumor drug can be employed.

In terms of a route of administration, it may be either systemic administration or local administration. The route of administration that is appropriate for a particular disease, symptomatic conditions, or other factors should be selected. As examples, parenteral administration including normal intravenous injection, intraarterial administration, subcutaneous administration, intracutaneous administration and intramuscular administration can be employed. Oral administration can be also employed. Further, transmucosal administration or dermal administration can be employed. When using the pharmaceutical composition for a neoplastic disease, direct administration to the neoplasm by an injection or the like is preferable.

In terms of an administration form, various forms can be selected in accordance with a treatment purpose. Typical examples thereof include an administration form of solid formulation such as a tablet, a pill, powder, powdered drug, fine granule, granule or a capsule, as well as an administration form of liquid formulation such as an aqueous formulation, ethanol formulation, suspension, fat emulsion, liposome formulation, clathrate such as cyclodextrin, syrup and an elixir. These can be further classified according to the administration route into oral formulation, parenteral formulation (drip injection formulation or injection formulation), nasal formulation, inhalant formulation, transvaginal formulation, suppositorial formulation, sublingual agents, eye drop formulation, ear drop formulation, ointment formulation, cream formulation, transdermal absorption formulation, transmucosal absorption formulation and the like, which can be respectively blended, formed and prepared according to conventional methods.

The present invention relates to a kit including at least one member selected from the group consisting of MAPKAPK3, a polynucleotide encoding MAPKAPK3, a vector containing the polynucleotide and a transformant containing the vector; and at least one member selected from the group consisting of TERT, a polynucleotide encoding TERT, a vector containing the polynucleotide and a transformant containing the vector. The kit can be used, for example, in the identification method of the present invention. Active MAPKAPK3 may be preferably used as MAPKAPK3.

The polynucleotide can be prepared from a human cDNA library by known genetic engineering techniques. A vector containing the polynucleotide and a transformant containing the vector can be obtained by introducing the polynucleotide using known genetic engineering techniques into a suitable expression vector, such as a vector derived from a bacterial plasmid, and by transfecting the obtained vector into a suitable cell by using a well-known method.

The aforementioned kit may include substances that are necessary for carrying out a determination, such as a signal and/or a marker for detecting the binding of MAPKAPK3 or active MAPKAPK3 to TERT, or the phosphorylation of TERT by active MAPKAPK3, buffer solutions and salts. The reagent kit may also include substances such as stabilizers and/or antiseptic agents. At the time of preparation, methods for preparation may be introduced in accordance with the respective substances to be used.

Hereinafter, the present invention may be explained more particularly with examples; however, the present invention is not limited to the following examples.

### Example 1

### (In-silico search for proteins having a function to interact with TERT)

The prediction of proteins having a function to interact with TERT that is a catalytic subunit of telomerase was conducted according to the method described in the pamphlet of International Publication No. WO 01/67299, as follows: i) decomposing the amino acid sequence of TERT into oligopeptides having a pre-determined length; ii) searching in a database for proteins having the amino acid sequence of each of the oligopeptides, or having homologous amino acid sequences to these amino acid sequences; iii) conducting local alignment between the proteins obtained and TERT; and iv) identifying proteins having a high local alignment score as a protein that might interact with TERT.

As a result of analysis (Fig. 1), MAPKAPK3 was identified as a protein being predicted to have a function to interact with TERT.

### Example 2

### (In vitro binding analysis of TERT with MAPKAPK3)

It was investigated whether TERT binds to MAPKAPK3 in an in vitro binding assay using a GST-pull down method. A MAPKAPK3, an active MAPKAPK3 and an inactive MAPKAPK3 were used as a MAPKAPK3.

### <Materials and their preparations>

### Construction of TERT expression plasmid

Human TERT cDNA was obtained from a human thymus gland cDNA (Clontech) by PCR. A TERT expression plasmid for animal cell was constructed by integrated human TERT cDNA into expression vector pCIneo (Promega) for animal cell.

### Construction of MAPKAPK3 expression plasmid

Human MAPKAPK3 cDNA was obtained from a human skeletal muscle cDNA (Clontech) by PCR. An expression plasmid of N-terminal GST fusion MAPKAPK3 for E. coli was constructed by integrating the human MAPKAPK3 cDNA into pGEX-4T (Amersham Bioscience) which is an expression vector of GST fusion protein for E. coli.

### Construction of expression plasmid of activated MAPKAPK3 and inactive MAPKAPK3

In order to construct an expression plasmid of N-terminal GST fusion active MAPKAPK3, a mutation has been introduced into the above-described MAPKAPK3 cDNA so that both threonine residues at positions 201 and 313 of the amino acid sequence of MAPKAPK3 are substituted with glutamic acid. An expression plasmid of N-terminal GST fusion active MAPKAPK3 was constructed by integrating the MAPKAPK3 cDNA, into which the mutation has been introduced, into pGEX-4T that is an expression vector of GST fusion protein for E. coli. In addition, in order to construct an expression plasmid of N-terminal GST fusion inactive MAPKAPK3, a mutation has been introduced into the above-described MAPKAPK3 cDNA so that both threonine residues at positions 201 and 313 of the amino acid sequence of MAPKAPK3 are substituted with alanine. An expression plasmid of N-terminal GST fusion inactive MAPKAPK3 was constructed by integrating the MAPKAPK3 cDNA, into which the mutation has been introduced, into pGEX-4T that is an expression vector of GST fusion protein for E. coli.

### Preparation of protein using each plasmid

TERT was synthesized in vitro as a ³⁵S-methionine labeled protein by utilizing TNT quick coupled transcription/translation systems (Promega).
GST fusion MAPKAPK3 (GST-MAPKAPK3), GST fusion active MAPKAPK3 (activated GST-MAPKAPK3) and GST fusion inactive MAPKAPK3 (inactivated GST-MAPKAPK3) was prepared for use by transfecting each expression plasmid that was constructed as described above into E. coli, followed purifiying with glutathione sepharose.

### Determination of the activity of each GST-MAPKAPK3

The phosphorylation reaction was performed by incubating 5 µg of GST-MAPKAPK3, activated GST-MAPKAPK3, inactivated GST-MAPKAPK3 or GST with 2 µg of HSP27 (Upstate) that was used as a substrate at 30°C for 15 minutes in 20 µl of phosphorylated buffer (25 mM Tris-HCl, pH7.5 / 5 mM β-glycerophophate / 2 mM dithiothreitol (DTT) / 0.1 mM Na₃VO₄ / 10mM MgCl₂ / 10 µM ATP) containing 5 µCi of [γ-³²P] ATP (3,000 Ci / mmol, PerkinElmer). Following the reaction, 20 µl of 2 x SDS sample buffer (4% SDS / 125 mM Tris HCl, pH6.8 / 20% glycerol / 0.01 % BPB / 10%β-mercaptoethanol) was added to the reaction solution, and treated at 100°C for 5 minutes. Subsequently, the protein was separated by 5-20% SDS-PAGE, followed by detecting phosphorylated HSP27 by an autoradiography using BAS2000 (Fuji Film).

### <Methods>

### Binding assay by GST-pull down method

20 µl of TERT synthesizing reaction solution together with 5 µg of GST-MAPKAPK3, activated GST-MAPKAPK3 or inactivated GST-MAPKAPK3 in 500 µl ofbinding buffer (20 mM Tris-HCl, pH8.0 / 140 mM NaCl / 1 mM ethylene glycol bis-tetraacetic acid (EGTA) / 1mM DTT /1% TritonX-100 / 10% glycerol) was left to stand on ice. Subsequently, 20 µl of glutathione sepharose 4B was added, and subjected to overturned mixing at 4°C overnight, followed by collecting the sepharose beads by centrifugation. After washing the sepharose beads four times with 500 µl of binding buffer, 20 µl of 2 x SDS sample buffer (125 mM Tris-HCl, pH6.8 / 4% SDS / 20% glycerol /0.01 % promophenol blue (BPB)) was added and boiled for 3 minutes, followed by separating the supernatant by 5 - 20% SDS-PAGE. Subsequently, TERT was detected by FLA3000 (Fuji Film). As a negative control, GST was used instead of each MAPKAPK3 to conduct the similar treatment as described above. Moreover, as a control for detection of TERT by SDS-PAGE, ³⁵S-methionine labeled TERT was used.

### <Results>

All of GST-MAPKAPK3, activated GST-MAPKAPK3 and inactivated GST-MAPKAPK3 bound to TERT in vitro (see Fig. 2). On the other hand, the binding of GST and TERT was not observed. From the results, it can be said that each GST-MAPKAPK3 specifically bound to TERT (see Fig. 2).

It has been thus clarified that all of GST-MAPKAPK3, activated GST-MAPKAPK3 and inactivated GST-MAPKAPK3 directly binds to TERT.

### Example 3

### (Binding analysis of TERT and MAPKAPK3 in a cell)

It was investigated whether TERT bind to MAPKAPK3 by a binding assay using a coexpression within a cell/immuno-coprecipitation method.

### <Materials>

### Construction of MAPKAPK3 expression plasmid

Human MAPKAPK3 cDNA was obtained by a RT-PCR, and integrated into pcDNA3.1 (+) (Invitrogen), an expression vector for animal cell. At the time, an expression plasmid of N-terminal HA-tagged MAPKAPK3 for animal cells was constructed by inserting HA-tag coding sequence at 5'-side of the cDNA.

The TERT expression plasmid prepared in Example 2 was used in this example.

### <Methods>

### Transfection and Binding assay by immuno-coprecipitation method

After culturing 4 x 10⁵ HEK293T cells at 37°C overnight under the atomosphere of 5% CO₂ (in a dish with 60 mm diameter), 2 µg of the expression plasmid of HA-tagged MAPKAPK3 or pcDNA3.1 (+) as a negative control was transfected together with 2 µg of TERT expression plasmid using FuGENE6 Transfection Reagent (Roche). After 2 days of culturing, cells were washed with ice-cold phosphate buffered physiologically saline (PBS (-)), collected and suspended in 500 µl of cell lysis buffer (20 mM Tris-HCl, pH7.4 / 150 mM NaCl / 1 mM ethylenediaminetetraacetic acid (EDTA) / 1 mM EGTA / 1% Triton X-100 / 2.5 mM sodium pyrophsphate) / 1 mM β-glycerophsphate / 1 mM Na₃VO₄ / protease inhibitor cocktail), and left to stand on ice for 10 minutes. Subsequently, centrifugation was conducted at 4°C for 10 minutes at 14,000 rpm to collect the supernatant for use as a cell lysate. Then, 10 µl of agarose-conjugated normal rabbit IgG (Sigma) was added to 500 µl of the cell lysate and subjected to overturned mixing at 4°C for 30 minutes, followed by subjecting to centrifugation to collect the supernatant. 10 µl of agarose-conjugated anti-HA antibody (Y-11, SantaCruz) was added to the collected supernatant, and subjected to overturned mixing at 4°C overnight, followed by subjecting to centrifugation to collect the agarose beads. After washing the agarose beads four times with 500 µl of a cell lysis buffer, 25 µl of 2 x SDS sample buffer was added thereto, and heated for 5 minutes, followed by separating the supernatant by 5 - 20% SDS-PAGE. Subsequently, the binding protein was detected by Western blotting using an anti-TERT antibody (L-20, SantaCruz). The detection was conducted using ECL Western blotting detection kit (Amersham Biosciences).

### <Results>

A band was observed by conducting immunoprecipitation of the cell lysate prepared from the cells in which TERT was co-expressed with HA-tagged MAPKAPK3 (HA-MAPKAPK3) with an anti-HA antibody followed by detecting by a Western blotting with an anti-TERT antibody gave a band (the lower panel of lane 2 in Fig. 3). On the other hand, any band was not observed by conducting immunoprecipitation of the cell lysate prepared from the cells in which only TERT was expressed followed by detecting by a Western blotting in the same manner as described above (the lower panel of the lane 1 in Fig. 3). The expression of HA-MAPKAPK3 and TERT in a cell was confirmed by Western blotting using an anti-HA antibody and an anti-TERT antibody, respectively (the upper panel and the middle panel of the lanes 1 and 2 in Fig. 3).

From these results, it has been clarified that TERT co-precipitates with HA-MAPKAPK3. Namely, it has been clarified that TERT bind to MAPKAPK3 in a cell.

### Example 4

### (Enhanced intracellular telomerase activity by transient expression of MAPKAPK3)

It was investigated whether intracellular telomerase activity varies by a transient expression of MAPKAPK3 in HEK293 cell that is a telomerase positive cell.

### <Materials>

The expression plasmid of N-terminal HA-tagged MAPKAPK3 for animal cell which was constructed in Example 3 was used for MAPKAPK3 expression plasmid. The expression plasmid of N-terminal HA-tagged active MAPKAPK3 was constructed based on the expression plasmid of N-terminal HA-tagged MAPKAPK3 by introducing a mutation so that both threonine residues at positions 201 and 313 of the amino acid sequence of MAPKAPK3 are substituted with glutamic acid. In addition, the expression plasmid of N-teminal HA-tagged inactive MAPKAPK3 was constructed based on the expression plasmid of N-terminal HA-tagged MAPKAPK3 by introducing a mutation so that both threonine residues at positions 201 and 313 of the amino acid sequence of MAPKAPK3 are substituted with alanine. For the negative control, pcDNA3.1 (+) was used.

### <Methods>

### Transfection and measurement of intracellular telomerase activity by TRAP method

After culturing 2 x 10⁴ HEK293T cells at 37°C overnight under the atomosphere of 5% CO₂ (24 well plate), 0.2 µg each of expression plasmid was transfected into the cells using FuGENE6 Transfection Reagent (Roche), respectively. After culturing for 48 hours, the cells were collected to measure the intracellular telomerase activity by a TRAP method using TRAPEZE XL Kit (Intergen).

Concretely, the cells was suspended in 100 µl of 1 x CHAPS cell lysis buffer (10 mM Tris-HCl, pH7.5 / 1 mM MgCl₂/ 1 mM EGTA / 0.1 mM benzamidine) / 5 mM β-mercaptoethanol / 0.5% CHAPS / 10% glycerol) and left to stand on ice for 30 minutes, followed by subjecting to centrifugation to collect the supernatant for use as a cell lysate. After measuring the protein concentration in the cell lysate by Coomassie Plus-200 Protein Assay Reagent (Pierce), TRAP reaction was performed using a cell lysate containing 5 ng of protein.

TRAP reaction was conducted by carrying out telomerase reaction at 30°C for 30 minutes, followed by amplifying by PCR the telomere repeat sequence generated by the telomerase reaction. PCR was performed by conducting 36 cycles (at 94°C for 30 seconds, at 59°C for 30 seconds, and at 72°C for one minute) followed by a reacting at 55°C for 25 minutes. Subsequently, the intensity of telomerase activity was determined by measuring a fluorescence intensity of the PCR reaction solution. The measurement of fluorescence intensity was carried out by measuring both fluorescence intensity of fluorescein that was labeled to PCR primer (which is an index for telomerase reaction product) and sulforhodamine (which is an index for internal standard) at measurement wavelength (λEx / λEm) of 485 nm / 535 nm and 585 nm / 620 nm, respectively, using fluorescence spectrophotometer F-2000 (Hitachi). At the time of measurement, 23 µl of reaction solution was diluted with 600 µl of measuring diluent (10 mM Tris-HCl, pH7.4 / 0.15 M NaCl / 2 mM MgCl₂) for measurement. Telomerase activity was represented by the ratio in respect to the internal standard (ΔFL / ΔRL).

### Determination of MAPKAPK3 activity by immune-complex kinase assay

After culturing 2 x 10⁵ HEK293T cell at 37°C overnight under the atomosphere of 5% CO₂ (6 cm dish), 2 µg of each expression plasmid was transfected to the cells using FuGENE6 Transfection Reagent (Roche). After culturing for 48 hours, cells were collected and suspended in 500 µl of cell lysis buffer (same component of the cell lysis buffer used in Example 3), and left to stand on ice for 20 minutes. Subsequently, centrifugation was conducted at 4°C for 10 minutes at 14,000 rpm to collect the supernatant for use as a cell lysate. Then, 10 µl of agarose-conjugated normal mouse IgG (Sigma) was added to 500 µl of the cell lysate and subjected to overturned mixing at 4°C for 30 minutes, followed by subjecting to centrifugation to collect the supernatant. 10 µl of anti-HA affinity Matrix (Roche) was added to the collected supernatant, and subjected to overturned mixing at 4°C for 2 hours, followed by subjecting to centrifugation to collect the agarose beads. Further, the agarose beads was washed with 500 µl of cell lysis buffer twice, and with 500 µl of kinase buffer (25 mM tris-HCl, pH7.5 / 5 mM β-glycerophsophate / 2 mM DTT / 0.1 mM Na₃VO₄ /10 mM MgCl₂) twice. Next, 20 µl of the kinase buffer containing 2 µg of HSP27 (Upstate), 10 µM ATP and 5 µCi of [γ-³²P] ATP (3,000 Ci /mmol, PerkinElmer) was added to the bead to conduct phosphorylation reaction at 30°C for 30 minutes. After the reaction, 20 µl of 2 x SDS sample buffer was added and boiled for 5 minutes, followed by separating the supernatant by a SDS-PAGE. The detection of the phosphorylated HSP27 was conducted by an autoradiography using FLA3000 (Fuji Film).

### <Results>

The enhanced intracellular telomerase activity was observed when an active MAPKAPK3 was transiently expressed in HEK293T cell that has the telomerase activity, comparing to the case that the negative control vector was transfected (about 1.4-fold increment, in Fig. 4). The enhanced intracellular telomerase activity by a transient expression of an active MAPKAPK3 was observed with a significance (p< 0.01) by student t-test. In contrast, the change of the intracellular telomerase activity was barely observed when MAPKAPK3 was transiently expressed in HEK293T.

On the other hand, the reduction of the intracellular telomerase activity was observed when an inactive MAPKAPK3 was transiently expressed in HEK293T cell (about 0.7-fold increment).

As a result of measurement of the kinase activity of each MAPKAPK3 that was transiently expressed in the cells by the phosphorylation assay, the kinase activity was observed only in an active MAPKAPK3.

From the above-described results, it was revealed that the telomerase activity of HEK293T cell was enhanced by transient expression of active MAPKAPK3, and that the effect MAPKAPK3 on enhancing the telomerase activity was dependent on the kinase activity of MAPKAPK3.

Moreover, it was revealed that the telomease activity of HEK293T cell is reduced by transient expression of an inactive MAPKAPK3. The inactive MAPKAPK3 binds to TERT (see Example 2), however, its kinase activity is reduced or deleted. From this finding, it is believed that an inactive MAPKAPK3 inhibits competitively the binding of intrinsic MAPKAPK3 to TERT and thereby inhibit the activation of telomerase, which results in the reduced telomerase activity.

### Example 5

### (Enhancement of the telomerase activity by MAPKAPK3/investigation in an in vitro system using HEK293 cell nuclear extract)

It has been reported that telomerase activity in the cell extract is reduced by the dephosphorylation treatment of the cell extract. From this fact, the influence of dephosphorylation treatment on the telomerase activity in a nuclear extract was investigated using a nuclear extract prepared from HEK293 cells. In addition, it was investigated whether the telomerase activity in a nuclear extract changes in response to the purified MAPKAPK3.

### <Materials>

### Preparation of MAPKAPK3 and determination of its activity

GST-MAPKAPK3, activated GST-MAPKAPK3 and inactivated GST-MAPKAPK3 were prepared by a method similar to the method described in Example 2. Moreover, the activity of each MAPKAPK3 was determined by the method described in Example 2.

### Preparation of the nucleus extracted solution from HEK293 cell

HEK293 cell was suspended in 400 µl of buffer A (10 mM Hepes-KOH, pH8.0 /10 mM KCl /0.1 mM EDTA / 0.1 mM EGTA / 1 mM DTT / protease inhibitor cocktail), and left to stand on ice for 15 minutes. After adding 25 µl of 10% Nonidet P-40 to the cell suspension, it was left to stand on ice for further 10 minutes. Subsequently, centrifugation was conducted to collect the precipitated nucleus at 4°C for 5 minutes at 14,000 rpm. 100 µl of buffer C (20 mM Hepes-KOH, pH8.0 /400 mM NaCl /1 mM EDTA/ 1 mM EGTA/ 1 mM DTT /protease inhibitor cocktail) was added to the precipitated nucleus and vigorously agitated, and left to stand on ice for 15 minutes, and then subjected to centrifugation at 4°C for 5 minutes at 14,000 rpm to collect supernatant for use as a nuclear extract. Subsequently, the buffer D (20 mM Hepes-KOH, pH8.0 /0.2 mM EDTA) was added to the collected nuclear extract to adjust the final concentration of NaCl in the nuclear extract to 150 mM. The nuclear extract was stored at -80°C until its use.

### <Methods>

### Dephosphorylation treatment of HEK293 cell nuclear extract and measurement of the telomerase activity in nucleus extracted solution

Dephosphorylation reaction was performed at 30°C for 30 minutes in 20 µl of reaction buffer (50 mM tris-HCl, pH8.0 / 1 mM MgCl₂) by adding 500, 100, 20, 4, 0.8, 0, munit of phosphatase, alkali-agarose (CIP-Agarose, Sigma) to HEK293 cell nuclear extract containing 2000 ng of protein. Subsequently, 1 *µ*l of the supernatant collected by centrifugation was diluted with the above-described reaction buffer to 20 *µ*l, 1 *µ*l of which was used for carrying out TRAP reaction to measure the telomerase activity.

Investigation of the influence of MAPKAPK3 on the telomerase activity in HEK293 cell nuclear extract.
After conducting dephosphorylation treatment of HEK293 cell nuclear extract by a method similar to the above-described method, 100, 50, 25, 12.5, 6.25 pmol of activated GST-MAPKAPK3 or GST was added to 1 µl of supernatant that was collected by centrifugation to carrying out phosphorylation reaction at 30°C for 30 minutes in 20 µl of reaction system (25 mM Tris-HCl, pH7.5 /5 mM β-glycerophosphate / 2 mM DTT / 0.1 mM Na₃VO₄ / 10mM MgCl₂ / 0.1 µM ATP). Subsequently, 1 µl of reaction solution was used for TRAP reaction to measure the telomerase activity. When MAPKAPK3 or an inactivated GST-MAPKAPK3 was used instead of an activated GST-MAPKAPK3, 100 pmol of the protein was used, respectively.

### <Results>

The reduction of telomerase activity was observed by dephosphorylation treatment in a dose dependent manner of phosphatase, alkali-agarose that is a dephosphorylation enzyme (Fig. 5). From the results, it was clarified that telomerase in HEK293 cell nuclear extract was activated depending upon phosphorylation.

Phosphorylation reaction was performed by adding an active MAPKAPK3 to the nuclear extract in which telomerase activity had been reduced by dephosphorylation treatment. As a result, telomerase activity was enhanced in a dose dependent manner of the active MAPKAPK3 (about 1.5-fold increment at maximum, Fig. 6). In contrast, the enhanced telomerase activity was not observed when phosphorylation reaction was performed similarly by adding GST. Moreover, the change of telomerase activity was not observed when phosphorylation reaction was performed similarly by adding MAPKAPK3 or inactive MAPKAPK3 (Fig. 7).

As a result of the measurement of the kinase activity of each purified by phosphorylation assay using HSP27 as a substrate, the kinase activity was observed only in an active MAPKAPK3.

From these results, it was revealed that MAPKAPK3 also enhanced the telomerase activity in vitro depending on its kinase activity.

### Example 6

### (Investigation of inhibition of telomerase activity and shortening oftelomere length by excessive expression of inactive MAPKAPK3)

It was investigated whether the telomerase activity is inhibited and whether telomere length is shortened by excessive expression of an inactive MAPKAPK3 in a cancer cell line.

The inactive MAPKAPK3 is believed to have the dominant negative effect and inhibits an intrinsic MAPKAPK3 action. Actually, the inactive MAPKAPK3 bound to TERT (see Example 2 and Fig. 2), and reduced telomerase activity in a cell when it was transiently expressed in HEK293T cell (see Example 4 and Fig. 4).

The inactive MAPKAPK3 was made to express excessively and stably in a cancer cell line by utilizing a recombinant retrovirus.

The measurement of telomerase activity was carried out by a TRAP method. Moreover, since it is considered that the inhibition of telomerase activity induces the shortened telomere length, the measurement oftelomere length in a cancer cell line in which an inactive MAPKAPK3 was excessively and stably expressed was conducted.

### <Materials and Methods>

### Construction of recombinant retrovirus vector for inactive MAPKAPK3 expression

A recombinant retrovirus vector for inactive MAPKAPK3 expression was constructed by integrating MAPKAPK3 cDNA (HA-tag coding sequence was inserted at 5' side) in which the codons for threonine residues at positions 201 and 313 (which are the phosphorylation site by p38) were both substituted with codon for alanine, into pQCXIP (Clontech) that is a retrovirus vector. The MAPKAKPK3 that is expressed by the vector is not activated since it is not phosphorylated by p38, thereby it dose not show any kinase activity. Moreover, the MAPKAPK3 that is expressed by the vector has HA-tag that is ligated to its N-terminal.

### Preparation of recombinant retrovirus

After culturing 2 x 10⁶ HEK293T cells at 37°C overnight under the atomosphere of 5% CO₂ (60 mm dish), 2 µg of the recombinant retrovirus vector for inactive MAPKAPK3 expression was transfected into the cells together with 2 µg of each pV Pack-GP and pV Pack-VSV-G (Stratagene) using FuGENE6 Transfection Reagent (Roche). After culturing for 2 days, the culture supernatant was collected, filtered with a 0.45 µm filter and used as a virus solution. For the negative control, pQCXIP without integrating inactive MAPKAPK3 cDNA was similarly treated as described above to prepare a virus solution (hereinafter referred to as empty virus).

### Infection of recombinant retrovirus to cultured cells

Human prostate cancer derived cell line PC-3 and human gliobrastoma derived cell line U-87MG were used as cancer cell lines. After culturing PC-3 and U-87MG overnight in 12 well plate, respectively (confluency of about 10%), the medium was replaced with a virus solution (350 µl /well) containing 8µg /ml of polybrene (Sigma). After culturing for 24 -36 hours, the medium was replaced with a medium containing 2.5 µg /ml of puromycin to start the selective cultivation of virus infected cells. After one week or more of the selective cultivation, detection of the expression protein was carried out by a Western blotting, and measurement oftelomerase activity was carried out by a TRAP method. The virus infected cells were kept in culture in a selective medium.

### Western blotting

The cells were suspended in SDS sample buffer (125 mM Tris-HCI, pH6.8 / 4% SDS / 20% glycerol / 0.01 % BPB), subjected to the supersonic treatment and boiled for 5 minutes, for use as a cell extract. After separating the cell extract by 5-20% SDS-PAGE, detection of the inactive MAPKAPK3 was carried out by Western blotting using an anti-HA polyclonal antibody (Y-11, Santa Cruz). In addition, detection of β-tubulin as a control was performed using an anti-β-tubulin polyclonal antibody (SantaCruz).

### Quantitative determination oftelomerase activity by TRAP method

The intracellular telomerase activity was measured by a method (TRAP method) similar to the method described in Example 4, using TRAPEZE XL Kit (Intergen). However, the cell lysis buffer comprising the following components was used: 10 mM Tris-HCl, pH7.5 / 1 mM MgCl₂ / 1 mM EGTA / 0.1 mM Benzamidine / 5 mM β-mercaptoethanol / 0.5% CHAPS /10% glycerol / 5 mM sodium pyrophosphate / 1 mM β-glycerophosphate / 1 mM Na₃VO₄ / 1 mM NaF. Further, a TRAP reaction was performed by utilizing a cell lysate containing 40 ng of protein.

### Extraction of genomic DNA from cultured cells

Genomic DNA was extracted from human gliobrastoma derived cell line U-87MG and used for measurement oftelomere length. Extraction of genomic DNA was performed with Blood & Cell Culture DNA Mini Kit (Qiagen) according to the user's manual attached to the kit.

### Measurement oftelomere length

The measurement oftelomere length was carried out by the analyzing terminal restriction fragments (TRF) method. Concretely, the measurement of telomere length was carried out using TeloTAGGG Telomere Length Assay Kit (Roche) according to the user's manual attached to the kit. Specifically, 2 µg of genomic DNA digested with HinfI and RsaI was separated by 0.8% agarose gel electrophoresis, and transferred onto a nylon membrane (positively-charged, Roche) by capillary transfer in the presence of 20 x SSC. Subsequently, the membrane has been baked at 120°C for 30 minutes to fix the DNA. Then, the membrane was prehybridized in DIG Easy Hyb solution that is included in the kit, followed by adding with digoxigenin (DIG) labeled telomere probe (included in the kit) to conduct hybridization at 42°C for 3 hours or more. After washing the membrane, the TRF signal was detected by a chemiluminescene method using alkalin phosphatase labeled anti-DIG antibody and CDP-Star (both are included in the kit).

### <Results>

Telomerase activity was significantly reduced in inactive MAPKAPK3 expression retrovirus infected cells (inactive 3pK) comparing to the telomerase activity in parent line or empty virus infected cell (Fig. 8-A). It was observed by a Western blotting that an inactive MAPKAPK3 was excessively expressed in inactive MAPKAPK3 expression retrovirus infected cells (inactive 3pK) (Fig. 8-B). Namely, the inactive MAPKAPK3 expression retrovirus infected cells were excessively and stably expressed an inactive MAPKAPK3.

The shortening of telomere length was observed in inactive MAPKAPK3 expression retrovirus infected cells in a time dependent manner (Fig. 9). On the 38^{th} day after the infection, the shortening oftelomere length was clearly observed comparing to parent line and empty virus infected cells (Fig. 9). On the other hand, the change of telomere length was not observed in the parent line and the empty virus infected cells (Fig. 9).

From these results, it was revealed that telomerase activity was inhibited by an excessive expression of the inactive MAPKAPK3, which resulted in the shortening oftelomere length.

The inactive MAPKAPK3 binds to TERT (see Example 2), but the kinase activity thereof is reduced or deleted. From this fact, it is believed that an inactive MAPKAPK3 inhibited the activation of telomerase by competitively inhibiting the binding of intrinsic MAPKAPK3 to TERT, and thereby induced the reduction of telomerase activity and the shortening of telomere length.

### INDUSTRIAL APPLICABILITY

According to the present invention, a method of identifying a compound that inhibits the telomerase activation or the telomerase activity can be provided. Furthermore, according to the present invention, the telomerase activation and the telomerase activity can be inhibited. Furthermore, the prevention and/or treatment of a disease attributable to the telomerase action or the enhanced activity of telomerase can be expected. Since telomerase is expressed in almost all of cancer cells regardless of the kinds of cancers, and is not expressed in a normal somatic cell except germ cell, it is believed that the present invention is effective, for example, for preventing and/or treating a variety of cancer diseases.

Thus, the present invention is useful for the basic research on the mechanisms of cell proliferation and cell senescence in which telomerase involves as well as on a disease attributable to the telomerase action and the enhanced activity of telomerase. Furthermore, the present invention is extremely useful for preventing and/or treating a disease attributable to the telomerase action and the enhanced activity of telomerase, for example, a cancer disease.

### SEQUENCE LISTING FREE TEXT

SEQ ID NO: 1: A polynucleotide encoding MAPKAPK3 (SEQ ID NO: 2)
SEQ ID NO: 3: A polynucleotide encoding TERT (SEQ ID NO: 4)
SEQ ID NO: 5: A polynucleotide encoding an inactive variant of MAPKAPK3 (SEQ ID: 2) which amino acid residues at positions 201 and 313 are both replaced to alanine from threonine
SEQ ID NO: 6: An inactive variant of MAPKAPK3 (SEQ ID NO: 2) which amino acid residues at positions 201 and 313 are both replaced to alanine from threonine
SEQ ID NO: 7: A polynucleotide encoding an active variant of MAPKAPK3 (SEQ ID NO: 2) which amino acid residues at positions 201 and 313 are both replaced to glutamic acid from threonine
SEQ ID NO: 8: An active variant of MAPKAPK3 (SEQ ID NO: 2) which amino acid residues at positions 201 and 313 are both replaced to glutamic acid from threonine
SEQ ID NO: 9: Partial sequence of TERT (SEQ ID NO: 4), which is highly homologous to that of MAPKAPK3 (SEQ ID NO: 2)
SEQ ID NO: 10: Partial sequence of MAPKAPK3 (SEQ ID NO: 2), which is highly homologous to that of TERT (SEQ ID NO: 4)
SEQ ID NO: 11: Partial sequence of TERT (SEQ ID NO: 4), which is highly homologous to that of MAPKAPK3 (SEQ ID NO: 2)
SEQ ID NO: 12: Partial sequence of MAPKAPK3 (SEQ ID NO: 2), which is highly homologous to that of TERT (SEQ ID NO: 4)
SEQ ID NO: 13: Partial sequence identical in the sequences of TERT (SEQ ID NO: 4) and MAPKAPK3 (SEQ ID NO: 2)

## Claims

1. A method for inhibiting the activation of telomerase, which is selected from the following group:
(i) a method for inhibiting the activation oftelomerase, comprising inhibiting the binding of MAPKAPK3 (mitogen-activated protein kinase-activated protein kinase-3) to TERT (telomerase reverse transcriptase); and
(ii) a method for inhibiting the activation oftelomerase, comprising inhibiting the phosphorylation of TERT by active MAPKAPK3.

2. A method for inhibiting the activation oftelomerase, comprising inhibiting the binding of MAPKAPK3 (mitogen-activated protein kinase-activated protein kinase-3) to TERT (telomerase reverse transcriptase).

3. A method for inhibiting telomerase activity, comprising using the method for inhibiting the activation of telomerase according to claim 1 or 2.

4. A method for inhibiting telomerase activity by an inactive variant of MAPKAPK3 (mitogen-activated protein kinase-activated protein kinase-3), wherein the variant is a variant that binds to TERT (telomerase reverse transcriptase).

5. The method for inhibiting telomerase activity according to claim 4, wherein the inactive variant of MAPKAPK3 (mitogen-activated protein kinase-activated protein kinase-3) is a protein shown by the amino acid sequence set forth in SEQ ID NO: 6 in the sequence listing.

6. A method for preventing and/or treating a disease attributable to the enhanced telomerase activity, comprising using the method for inhibiting the activation oftelomerase according to claim 1 or 2 and/or the method for inhibiting telomerase activity according to any one of claims 3 to 5.

7. The method for preventing and/or treating a disease attributable to the enhanced telomerase activity according to claim 6, wherein the disease attributable to the enhanced telomerase activity is a cancer disease.

8. The method for preventing and/or treating a disease attributable to the enhanced telomerase activity according to claim 7, wherein the cancer disease is any of breast cancer, renal cell carcinoma, acute leukemia, glia cell carcinoma, prostatic cancer, neuroepithelial carcinoma, squamous cell carcinoma, liver cell carcinoma, prostatic cancer, and non-small cell lung cancer.

9. The method for preventing and/or treating a disease attributable to the enhanced telomerase activity according to claim 7, wherein the cancer disease is a breast cancer disease.

10. A method of identifying a compound that inhibits the binding of MAPKAPK3 (mitogen-activated protein kinase-activated protein kinase-3) to TERT (telomerase reverse transcriptase), wherein the method comprises contacting a compound with MAPKAPK3 and/or TERT under a condition allowing the compound to interact with MAPKAPK3 and/or the TERT, introducing a system using a signal and/or a marker that is generated by the binding of MAPKAPK3 to TERT, and detecting the presence, absence or change of the signal and/or the marker, thereby determining whether the compound inhibits the binding of MAPKAPK3 to the TERT.

11. A method of identifying a compound that inhibits the phosphorylation of TERT (telomerase reverse transcriptase) by active MAPKAPK3 (mitogen-activated protein kinase-activated protein kinase-3), wherein the method comprises contacting a compound with active MAPKAPK3 and/or TERT under a condition allowing the compound to interact with active MAPKAPK3 and/or TERT, introducing a system using a signal and/or a marker that is generated by the phosphorylation of TERT by active MAPKAPK3, detecting the presence, absence or change of the signal and/or the marker, thereby determining whether the compound inhibits the phosphorylation of TERT by active MAPKAPK3.

12. An agent for inhibiting the activation of telomerase, which is selected from the following group:
(i) an agent for inhibiting the activation of telomerase, comprising inhibiting the binding of MAPKAPK3 (mitogen-activated protein kinase-activated protein kinase-3) to TERT (telomerase reverse transcriptase); and
(ii) an agent for inhibiting the activation of telomerase, comprising inhibiting the phosphorylation of TERT by active MAPKAPK3.

13. An agent for inhibiting the activation oftelomerase, which is selected from the following group:
(i) an agent for inhibiting the activation oftelomerase, comprising at least one compound that inhibits the binding of MAPKAPK3 (mitogen-activated protein kinase-activated protein kinase-3) to TERT (telomerase reverse transcriptase); and
(ii) an agent for inhibiting the activation of telomerase, comprising at least one compound that inhibits the phosphorylation of TERT by active MAPKAPK3.

14. An agent for inhibiting telomerase activity, which is selected from the following group:
(i) an agent for inhibiting telomerase activity, comprising inhibiting the binding of MAPKAPK3 (mitogen-activated protein kinase-activated protein kinase-3) to TERT (telomerase reverse transcriptase); and
(ii) an agent for inhibiting telomerase activity, comprising inhibiting the phosphorylation of TERT by active MAPKAPK3.

15. An agent for inhibiting telomerase activity, which is selected from the following group:
(i) an agent for inhibiting telomerase activity, comprising at least one compound that inhibits the binding of MAPKAPK3 (mitogen-activated protein kinase-activated protein kinase-3) to TERT (telomerase reverse transcriptase); and
(ii) an agent for inhibiting telomerase activity, comprising at least one compound that inhibits the phosphorylation of TERT by active MAPKAPK3.

16. An agent for inhibiting telomerase activity comprising an inactive variant of MAPKAPK3 (mitogen-activated protein kinase-activated protein kinase-3), wherein the variant is a variant that binds to TERT (telomerase reverse transcriptase).

17. The agent for inhibiting telomerase activity comprising an inactive variant of MAPKAPK3 (mitogen-activated protein kinase-activated protein kinase-3) according to claim 16, wherein the inactive variant of MAPKAPK3 is a protein shown by the amino acid sequence set forth in SEQ ID NO: 6 in the sequence listing.

18. An agent for preventing and/or treating a disease attributable to the enhanced telomerase activity, comprising using the method for inhibiting the activation of telomerase according to claim 1 or 2 and/or the method for inhibiting telomerase activity according to any one of claims 3 to 5.

19. An agent for preventing and/or treating a disease attributable to the enhanced telomerase activity, comprising at least one of the agent for inhibiting the activation of telomerase according to claim 12 or 13 and the agent for inhibiting telomerase activity according to any one of claim 14 to 17.

20. The agent for preventing and/or treating a disease attributable to the enhanced telomerase activity according to claim 18 or 19, wherein the disease attributable to the enhanced telomerase activity is a cancer disease.

21. The agent for preventing and/or treating a disease attributable to the enhanced telomerase activity according to claim 20, wherein the cancer disease is any of breast cancer, renal cell carcinoma, acute leukemia, glia cell carcinoma, prostatic cancer, neuroepithelial carcinoma, squamous cell carcinoma, liver cell carcinoma, prostatic cancer, and non-small cell lung cancer.

22. The agent for preventing and/or treating a disease attributable to the enhanced telomerase activity according to claim 20, wherein the cancer disease is a breast cancer disease.

23. A reagent kit, comprising at least one selected from the group consisting of MAPKAPK3 (mitogen-activated protein kinase-activated protein kinase-3), a polynucleotide encoding MAPKAPK3, a vector containing the polynucleotide, and a transformant containing the vector; and at least one selected from the group consisting of TERT (telomerase reverse transcriptase), a polynucleotide encoding TERT, a vector containing the polynucleotide, and a transformant containing the vector.
